# EUROPEAN PATENT APPLICATION

(11) **EP 4 650 369 A1**
(43) Date of publication of application: **19.11.2025**
(21) Application number: 24741674.6
(22) Date of filing: 09.01.2024
(51) Int. Cl.: C07K 16/28, A61P 43/00, A61P 11/00, C12N 15/113, A61K 31/713, A61K 39/00

(54) **FIBROSIS TREATMENT CONTAINING ICAM-1 INHIBITOR**

(30) Priority: 10.01.2023 KR 20230003390; 16.10.2023 KR 20230137910
(71) Applicant: FNCTBiotech Inc., Seoul 04626 (KR)
(72) Inventor: JIN, Young-Woo, Seongnam-si, Gyeonggi-do 13589 (KR); LEE, Su Jae, Seoul 03168 (KR); KIM, Min Jung, Seoul 01812 (KR)
(74) Representative: Dompatent
(86) International application number: PCT/KR2024/000417
(87) International publication number: WO 2024/151053

(57) **Abstract**

Disclosed herein is a fibrosis treatment containing an ICAM-1 inhibitor. It has been discovered that inhibiting the function of ICAM-1 in tissues that have been affected with fibrosis results in the restoration of the fibrotic tissue to normal tissue and the treatment of fibrosis, and through research, the treatment mechanism was elucidated. The fibrosis treatment containing an ICAM-1 inhibitor can inhibit ICAM-1 mediated signaling, and consequently, suppresses epithelial to mesenchymal transition (EMT) of tissue epithelial cells in which ICAM-1 mediated signaling is involved and/or the accumulation of extracellular matrix in which ICAM-1 mediated signaling is involved. The fibrosis treatment containing an ICAM-1 inhibitor has the therapeutic mechanism of inhibiting the induction of EMT in tissue epithelial cells, preventing the differentiation into myofibroblasts through inhibition of the activity of local fibroblasts, and/or reducing myofibroblasts by directly acting thereon.

## Description

### Technical Field

The present disclosure relates to fibrosis and related diseases treatment, and a method of treating the same.

### Background Art

Fibrosis is a condition characterized by an excessive accumulation of extracellular matrix, in particular collagen, in tissues. When fibrosis develops, affected tissues lose their normal function, and become rigid due to accumulation of extracellular matrix. It is known that fibrosis can develops in almost all tissues of a body, and fibrotic changes in various tissues can lead to a wide range of diseases, disorders, and/or pathological conditions. Fibrosis may result from persistent stimulation or severe injury to tissues, and is also a common and final pathological outcome of many chronic inflammatory diseases.

Meanwhile, InterCellular Adhesion Molecule-1 (ICAM-1), also referred to as Cluster of Differentiation 54 (CD54), is an intercellular adhesion molecule or proteins belonging to an immunoglobulin superfamily that includes antibodies and T-cell receptors, etc. ICAM-1 is a transmembrane protein primarily expressed on leukocytes and endothelial cells, functioning as an intercellular adhesion molecule, and is known to be able to bind to lymphocyte function-associated antigen 1 (LFA-1), a receptor expressed on leukocytes. ICAM-1 is activated by secretion of cytokines such as interleukin-1 (IL-1) and tumor necrosis factor (TNF) in tissues, and when activated on endothelial cells of blood vessels, ICAM-1 binds to LFA-1 of leukocytes, captures the leukocytes, and transports them into tissues. Furthermore, ICAM-1 is associated with immune responses, and is also known to play a role in intercellular signaling.

### Disclosure

### Technical Problem

The present disclosure aims to elucidate a therapeutic mechanism for fibrosis related to inhibition of ICAM-1, and further aims to provide a method of treating fibrosis, a fibrosis treatment, and use of substances associated therewith.

### Technical Solution

In order to solve the above technical problem, the present disclosure provides a fibrosis treatment containing an ICAM-1 inhibitor, use thereof, and a method of treating fibrosis using the ICAM-1 inhibitor. The present inventors have revealed that inhibition of ICAM-1 in fibrotic tissues results in suppression of epithelial-to-mesenchymal transition (EMT), inhibition of extracellular matrix (ECM) accumulation, and reduction of myofibroblasts, thereby treating fibrosis. Furthermore, these effects are accomplished regardless of types of fibrotic tissues, indicating that the above-mentioned therapeutic mechanism may be effective for all types of fibrosis. The present disclosure provides an ICAM-1 neutralizing antibody, and an ICAM-1 gene-targeting siRNA, as a representative means of inhibiting ICAM-1, and demonstrates the therapeutic effect on fibrosis by the ICAM-1 neutralizing antibody and the ICAM-1 gene-targeting siRNA.

### Advantageous Effects

According to the present disclosure, an ICAM-1 inhibitor, and a method of treating fibrosis using the same can prevent or treat fibrosis regardless of types of fibrotic tissues.

### Description of Drawings

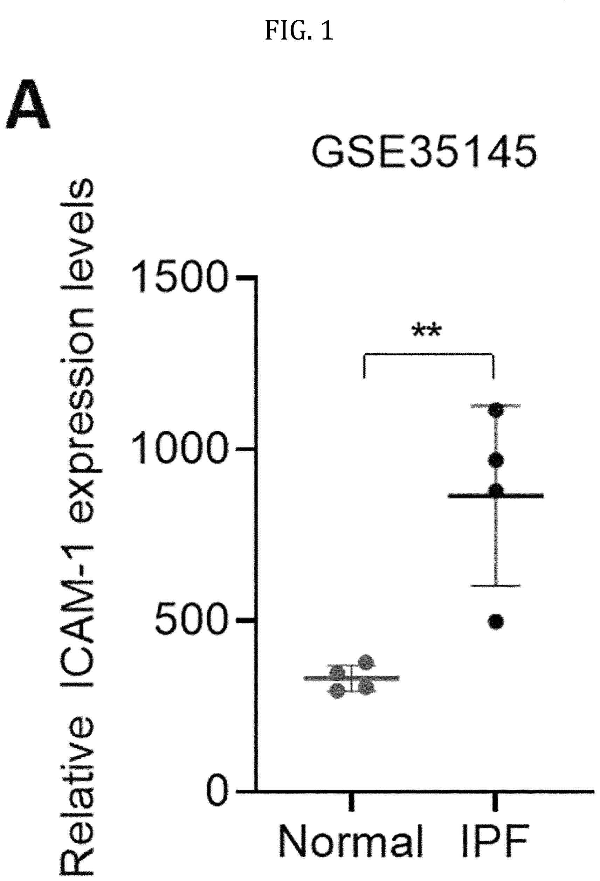

FIG. 1 shows analysis results of ICAM-1 gene expression in pulmonary tissues from patients with idiopathic pulmonary fibrosis (IPF), and normal pulmonary tissues, based on data from NCBI database. Here, "Normal" refers to normal pulmonary tissues, and "IPF" refers to tissues from patients with idiopathic pulmonary fibrosis.

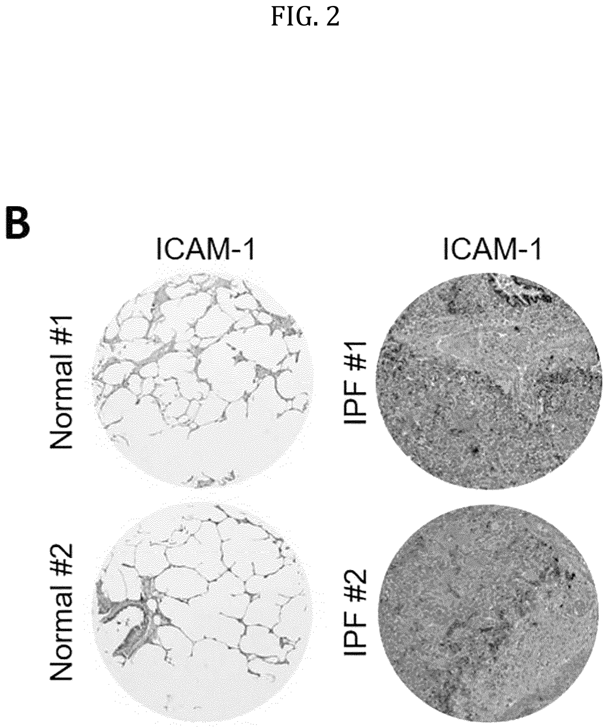

FIG. 2 shows results of staining an ICAM-1 protein in pulmonary tissue samples from patients with idiopathic pulmonary fibrosis (IPF), and from normal pulmonary tissue samples, followed by observation of images of these tissues under a microscope. Here, "Normal #1" and "Normal #2" refer to normal pulmonary tissues, while "IPF #1" and "IPF #2" refer to pulmonary tissues from patients with idiopathic pulmonary fibrosis.

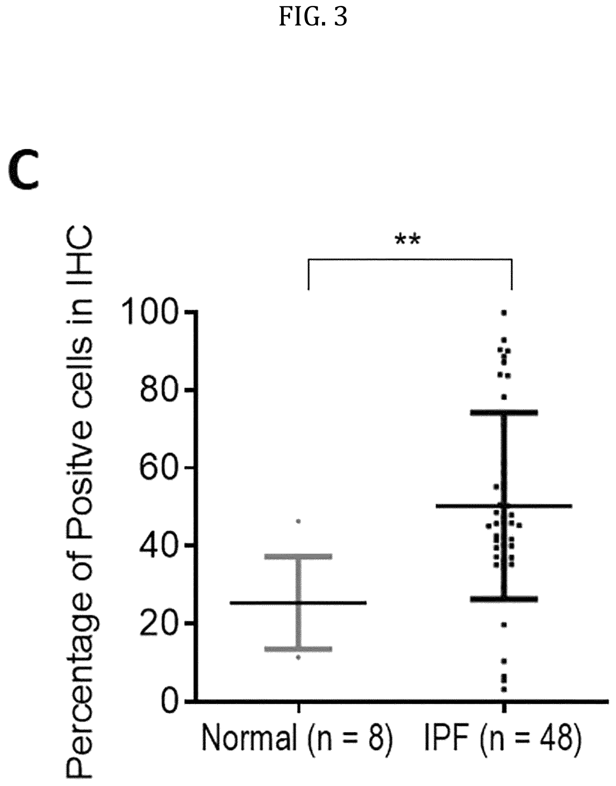

FIG. 3 shows plotted results of measuring percentage of IHC-positive cells in pulmonary tissue samples from patients with idiopathic pulmonary fibrosis (IPF), and in normal pulmonary tissue samples. Here, "Normal" refers to normal pulmonary tissues, and "IPF" refers to tissues from patients with idiopathic pulmonary fibrosis.

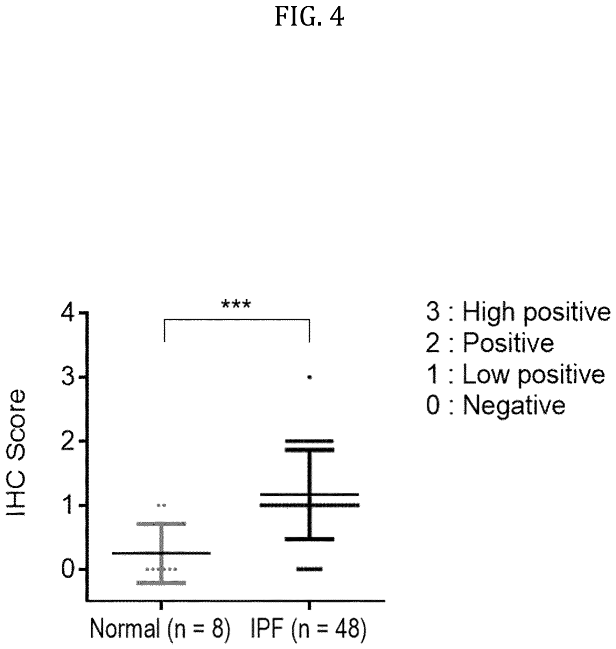

FIG. 4 shows plotted IHC scores calculated for pulmonary tissue samples from IPF patients (IPF), and normal pulmonary tissue samples. Here, "Normal" refers to normal pulmonary tissues, and "IPF" refers to tissues from idiopathic pulmonary fibrosis patients.

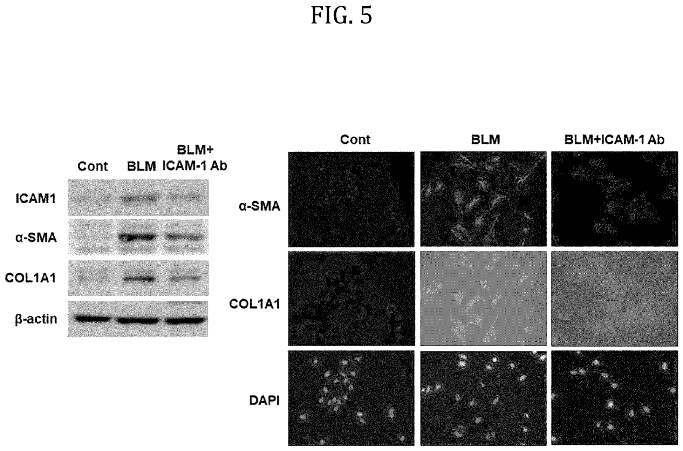

FIG. 5 shows results of Experimental Example 3.1, in which BEAS2B cells, a normal pulmonary epithelial cell line, was administered with PBS, bleomycin (BLM), and ICAM-1 antibody, followed by analyzing expression levels of α-SMA, Col1A1, and ICAM-1 proteins 36 hours after administration. A left panel shows results of analysis of protein expression using Western blotting, while a right panel shows results of immunofluorescence staining observed under a fluorescence microscope. Here, "Cont" refers to a group administered with PBS only, "BLM" refers to a group administered with bleomycin only, and "BLM + ICAM-1 Ab" refers to a group co-administered with both bleomycin and an ICAM-1 antibody.

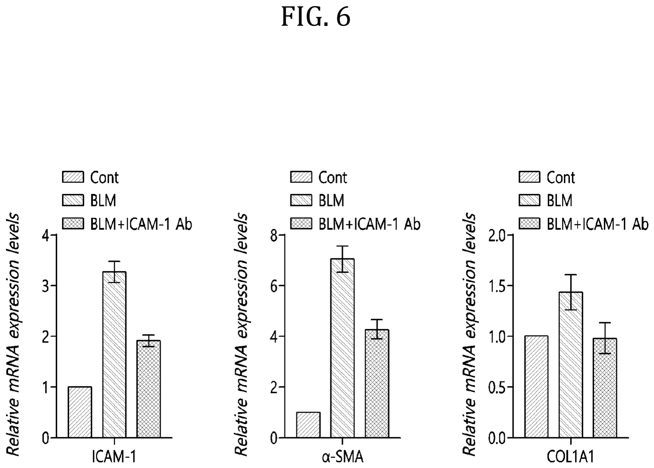

FIG. 6 shows results of Experimental Example 3.2, in which BEAS2B cells, a normal pulmonary epithelial cell line were administered with PBS, bleomycin (BLM), or ICAM-1 antibody, followed by analyzing expression levels of α-SMA, Col1A1, and ICAM-1 proteins genes 36 hours after administration. In each graph, "Cont" refers to a group administered with PBS only, "BLM" refers to a group administered with bleomycin only, and "BLM + ICAM-1 Ab" refers to a group co-administered with both bleomycin and ICAM-1 antibody.

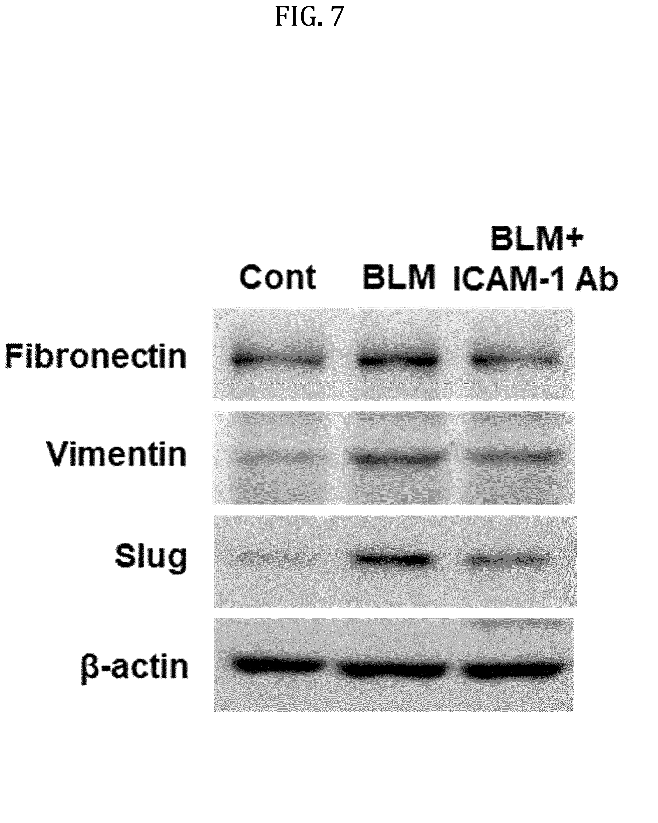

FIG. 7 shows results of Experimental Example 3.3, in which BEAS2B cells, a normal pulmonary epithelial cell line, was administered with PBS, bleomycin (BLM), and ICAM-1 antibody, followed by analyzing expression levels of Fibronectin, Vimentin, and Slug proteins using Western blotting 36 hours after administration. Here, "Cont" refers to a group administered with PBS only, "BLM" refers to a group administered with bleomycin only, and "BLM + ICAM-1 Ab" refers to a group co-administered with both bleomycin and ICAM-1 antibody.

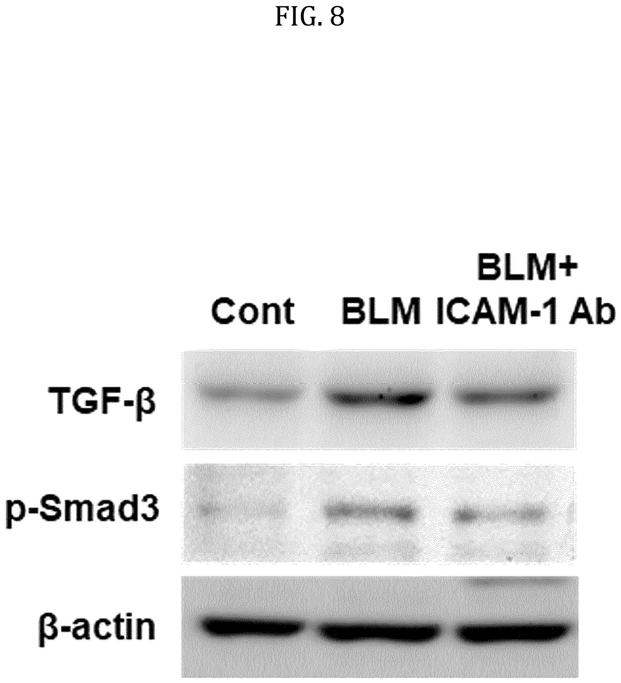

FIG. 8 shows results of Experimental Example 3.4, in which BEAS2B cells, a normal pulmonary epithelial cell line, was administered with PBS, bleomycin (BLM), and ICAM-1 antibody, followed by analyzing expression level of TGF-β, and its downstream signaling proteins, activation of Smad3 (p-Smad3), using Western blotting 36 hours after administration. Here, "Cont" refers to a group administered with PBS only, "BLM" refers to a group administered with bleomycin only, and "BLM + ICAM-1 Ab" refers to a group co-administered with both bleomycin and ICAM-1 antibody.

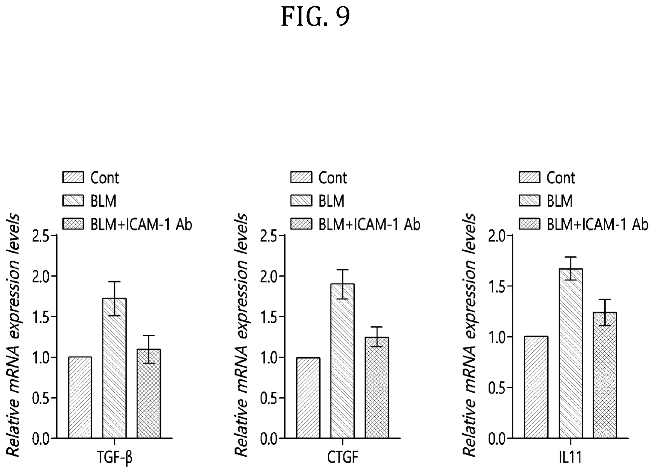

FIG. 9 shows results of Experimental Example 3.5, in which BEAS2B cells, a normal pulmonary epithelial cell line, was administered with PBS, bleomycin (BLM), and ICAM-1 antibody, followed by measuring expression levels of TGF-β, and CTGF genes 36 hours after administration. Here, "Cont" refers to a group administered with PBS only, "BLM" refers to a group administered with bleomycin only, and "BLM + ICAM-1 Ab" refers to a group co-administered with both bleomycin and ICAM-1 antibody.

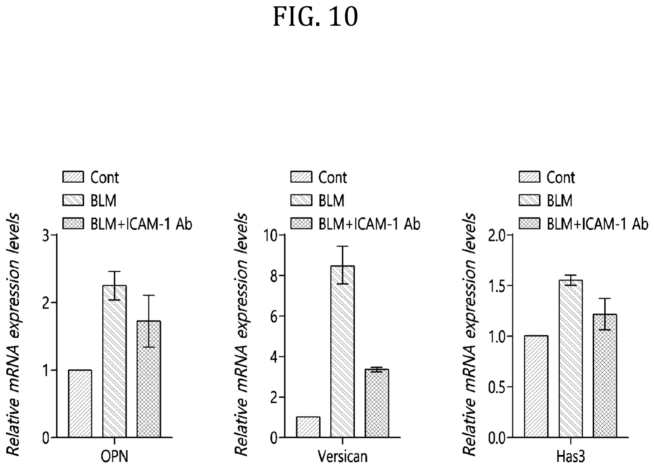

FIG. 10 shows results of Experimental Example 3.6, in which BEAS2B cells, a normal pulmonary epithelial cell line, was administered with PBS, bleomycin (BLM), and an ICAM-1 antibody, followed by measuring expression levels of extracellular matrix osteopontin (OPN), versican, and Has3 genes 36 hours after administration. Here, "Cont" refers to a group administered with PBS only, "BLM" refers to a group administered with bleomycin only, and "BLM + ICAM-1 Ab" refers to a group co-administered with both bleomycin and ICAM-1 antibody.

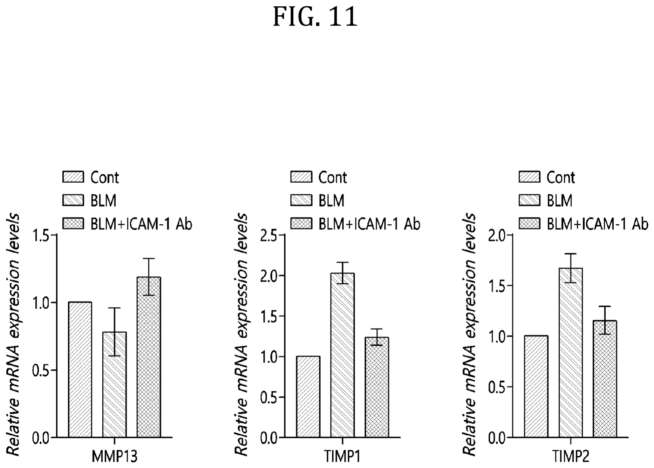

FIG. 11 shows results of Experimental Example 3.6, in which BEAS2B cells, a normal pulmonary epithelial cell line, was administered with PBS, bleomycin (BLM), and ICAM-1 antibody, followed by measuring expression levels of collagenase, MMP13, and extracellular matrix degrading enzyme inhibitors, TIMP1, TIMP2. Here, "Cont" refers to a group administered with PBS only, "BLM" refers to a group administered with bleomycin only, and "BLM + ICAM-1 Ab" refers to a group co-administered with both bleomycin and ICAM-1 antibody.

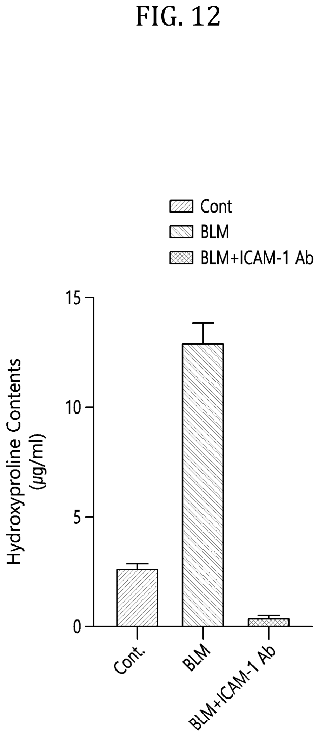

FIG. 12 shows results of Experimental Example 3.7, in which BEAS2B cells, a normal pulmonary epithelial cell line, was administered with PBS, bleomycin (BLM), and ICAM-1 antibody, followed by analyzing changes in Hydroxyproline content. Here, "Cont" refers to a group administered with PBS only, "BLM" refers to a group administered with bleomycin only, and "BLM + ICAM-1 Ab" refers to a group co-administered with both bleomycin and ICAM-1 antibody.

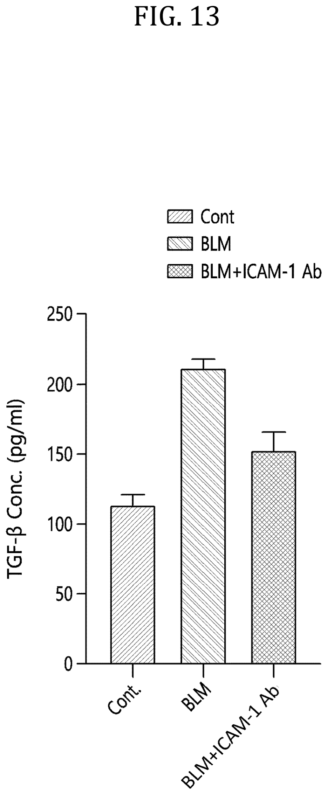

FIG. 13 shows results of Experimental Example 3.8, in which BEAS2B cells, a normal pulmonary epithelial cell line, was administered with PBS, bleomycin (BLM), and ICAM-1 antibody, followed by analyzing changes in TGF-β concentration. Here, "Cont" refers to a group administered with PBS only, "BLM" refers to a group administered with bleomycin only, and "BLM + ICAM-1 Ab" refers to a group co-administered with both bleomycin and ICAM-1 antibody.

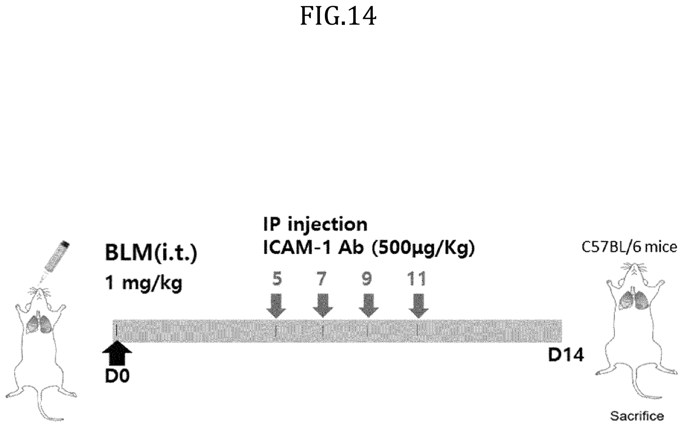

FIG. 14 schematically illustrates an administration schedule of bleomycin and ICAM-1 antibody in a bleomycin-induced pulmonary fibrosis animal model, according to Experimental Example 4.1.

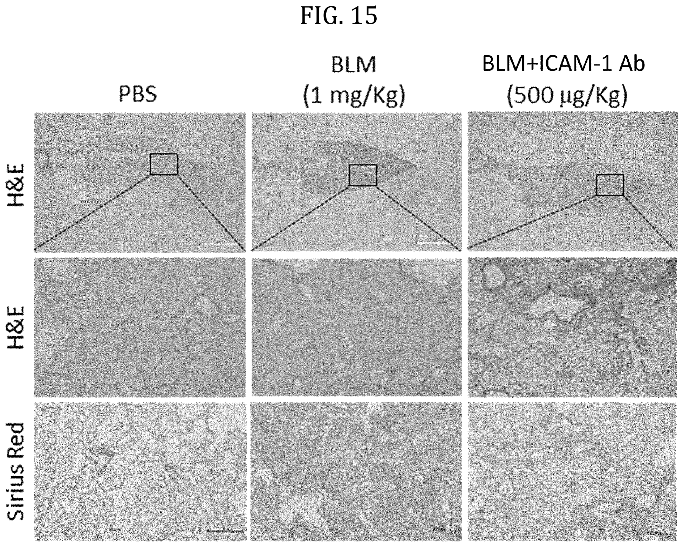

FIG. 15 shows results of H&E and Sirius Red staining of pulmonary tissues on day 14 from representative samples in each group administered with PBS, BLM, and an ICAM-1 Ab, followed by observing stained pulmonary tissues, according to Experimental Example 4.2. Here, "PBS" refers to a group administered with PBS only, "BLM" refers to a group administered with bleomycin only, and "BLM + ICAM-1 Ab" refers to a group administered with bleomycin followed by an ICAM-1 antibody.

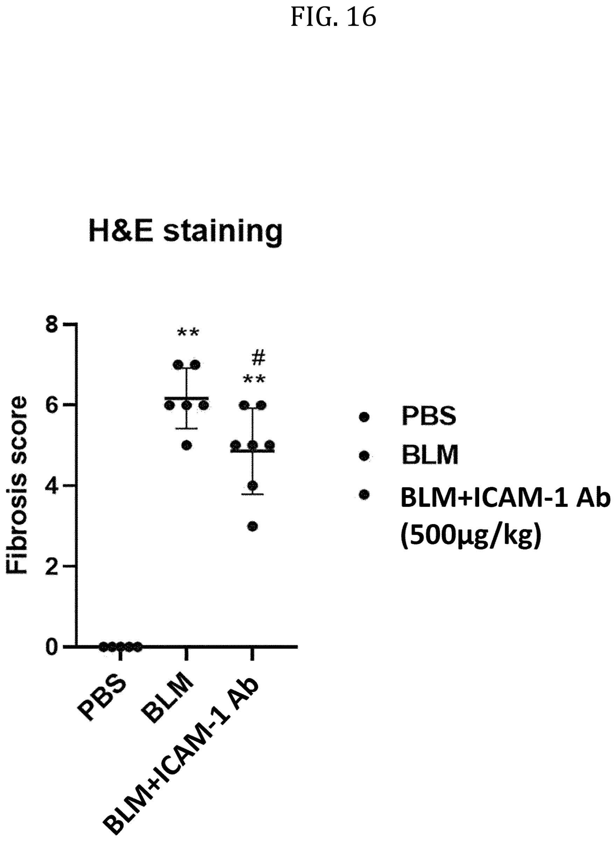

FIG. 16 shows results of H&E staining of pulmonary tissues on day 14 from representative samples in each group administered with PBS, BLM, and ICAM-1 Ab, followed by measuring Fibrosis Scores of stained pulmonary tissues, according to Experimental Example 4.2. Here, "PBS" refers to a group administered with PBS only, "BLM" refers to a group administered with bleomycin only, and "BLM + ICAM-1 Ab" refers to a group administered with bleomycin followed by an ICAM-1 antibody.

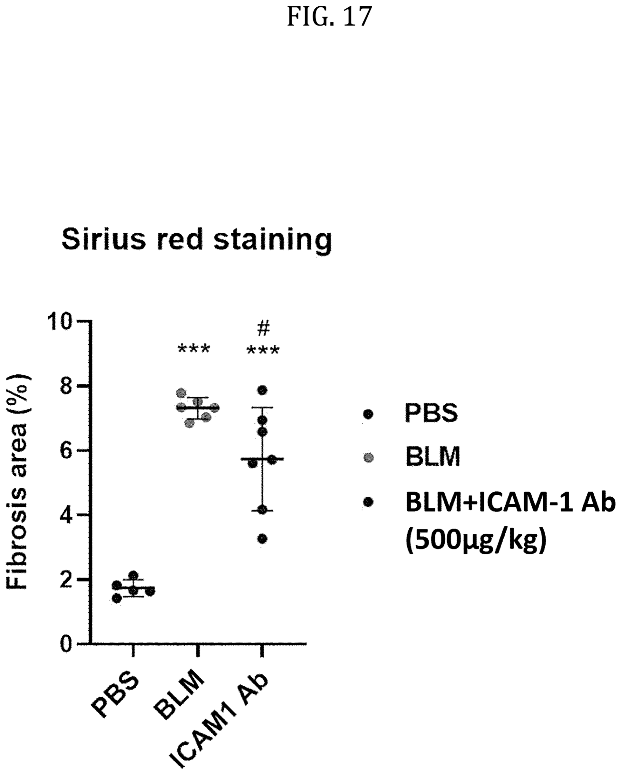

FIG. 17 shows results of Sirius Red staining of pulmonary tissues on day 14 from representative samples in each group administered with PBS, BLM, and ICAM-1 Ab, followed by measuring Fibrosis Areas of stained pulmonary tissues, according to Experimental Example 4.2. Here, "PBS" refers to a group administered with PBS only, "BLM" refers to a group administered with bleomycin only, and "BLM + ICAM-1 Ab" refers to a group administered with bleomycin followed by an ICAM-1 antibody.

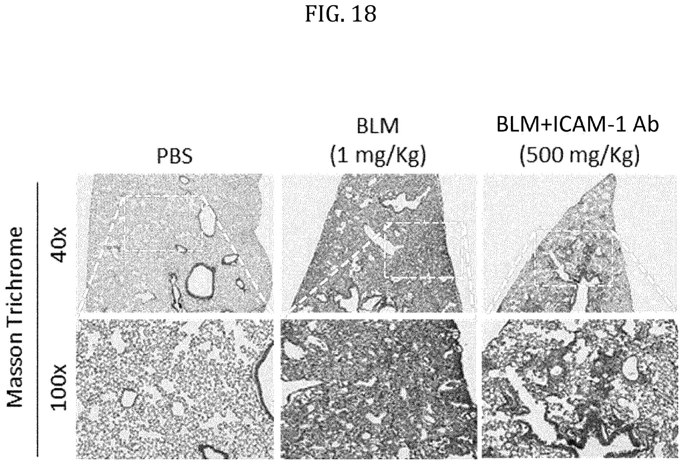

FIG. 18 shows observation results of Masson's trichrome staining performed to assess collagen accumulation in pulmonary tissues on day 14 from representative samples in each group administered with PBS, BLM, and ICAM-1 Ab, according to Experimental Example 4.2. Here, "PBS" refers to a group administered with PBS only, "BLM" refers to a group administered with bleomycin only, and "BLM + ICAM-1 Ab" refers to a group administered with bleomycin followed by an ICAM-1 antibody.

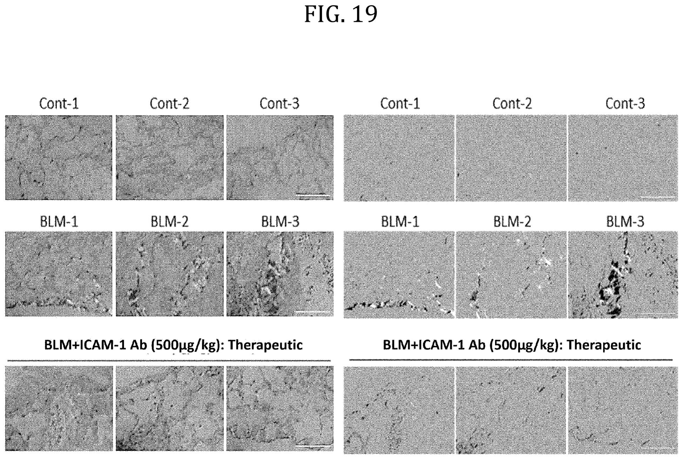

FIG. 19 shows observation results of polarized microscopy performed to assess collagen cross-linking in pulmonary tissues on day 14 from representative samples in each group administered with PBS, BLM, and ICAM-1 Ab, according to Experimental Example 4.2. Here, "Cont-1", "Cont-2", and "Cont-3" refers to groups administered with PBS only, "BLM-1", "BLM-2", and "BLM-3" refers to groups administered with bleomycin only, and "BLM + ICAM-1 Ab" refers to a group administered with bleomycin followed by an ICAM-1 antibody.

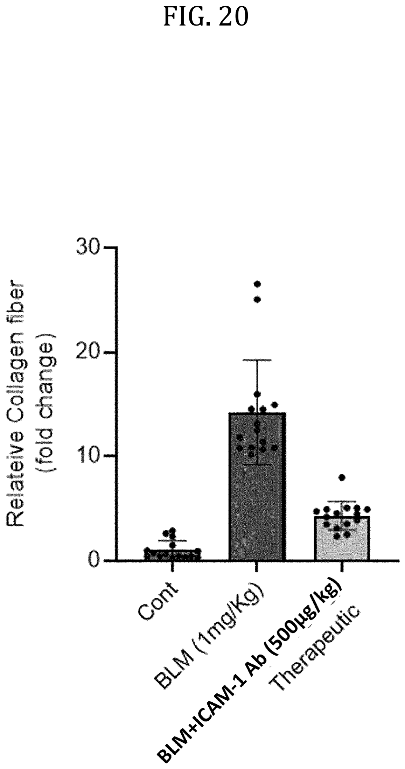

FIG. 20 shows results of analyzing collagen fiber content of pulmonary tissues on day 14 from representative samples in each group administered with PBS, BLM, and ICAM-1 Ab, according to Experimental Example 4.2. Here, "Cont" refers to a group administered with PBS only, "BLM" refers to a group administered with bleomycin only, and "BLM + ICAM-1 Ab" refers to a group administered with bleomycin followed by an ICAM-1 antibody.

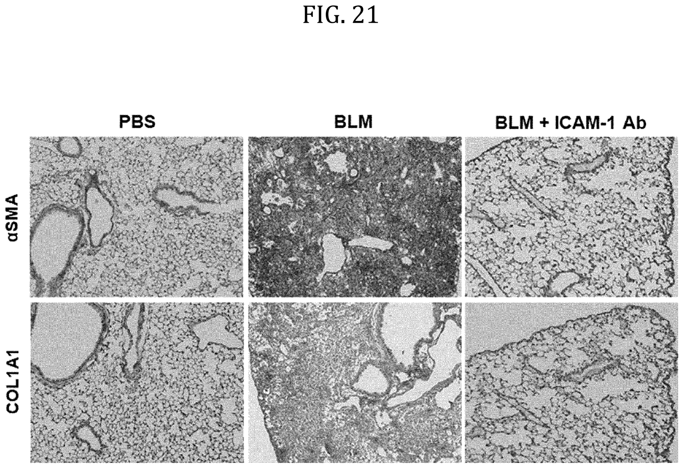

FIG. 21 shows results of immunohistochemical staining for α-SMA and Col1A1 proteins of pulmonary tissues on day 14 from representative samples in each group administered with PBS, BLM, and ICAM-1 Ab-according to Experimental Example 4.3, wherein these proteins were stained brown to observe whether these proteins were accumulated or not. Here, "PBS" refers to a group administered with PBS only, "BLM" refers to a group administered with bleomycin only, and "BLM + ICAM-1 Ab" refers to a group administered with bleomycin followed by an ICAM-1 antibody.

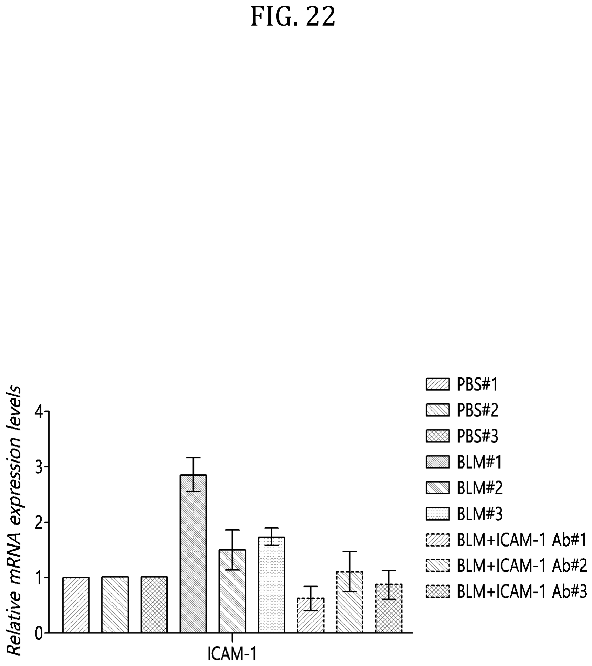

FIG. 22 shows results of analyzing expression level of an ICAM-1 gene in pulmonary tissues on day 14 from representative samples in each group administered with PBS, BLM, and ICAM-1 Ab, according to Experimental Example 4.4. Here, "PBS #1" to "PBS #3" refer to individual 1 to 3 of a group administered with PBS only, "BLM #1" to "BLM #3" refer to individual 1 through 3 of a group administered with bleomycin only, and "BLM + ICAM-1 Ab #1" to "BLM + ICAM-1 Ab #3" refer to individual 1 through 3 of a group administered with an ICAM-1 antibody after bleomycin-induced fibrosis developed.

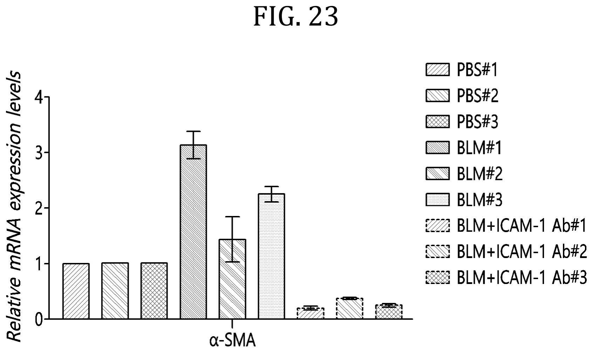

FIG. 23 shows results of analyzing expression levels of α-SMA gene in pulmonary tissues on day 14 from representative samples in each group administered with PBS, BLM, and ICAM-1 Ab, according to Experimental Example 4.4. Here, "PBS #1" to "PBS #3" refer to individual 1 to 3 of a group administered with PBS only, "BLM #1" to "BLM #3" refer to individual 1 to 3 of a group administered with bleomycin only, and "BLM + ICAM-1 Ab #1" to "BLM + ICAM-1 Ab #3" refer to individual 1 to 3 of a group administered with ICAM-1 antibody after bleomycin-induced fibrosis developed.

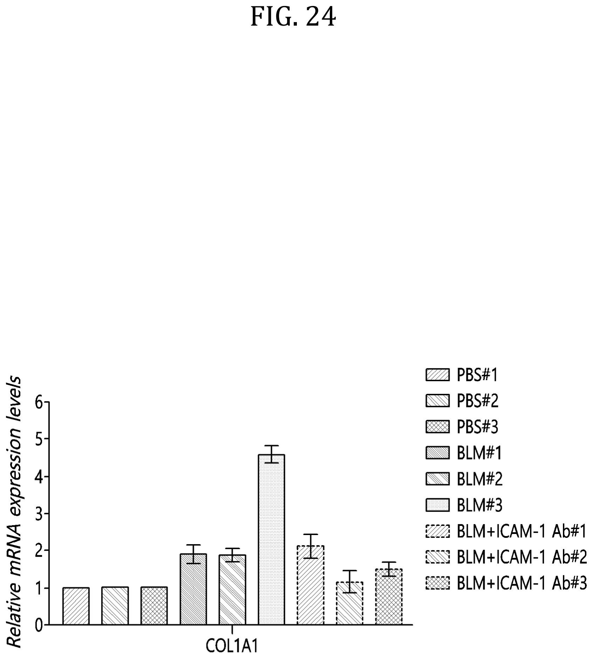

FIG. 24 shows results of analyzing expression level of COL1A1 gene in pulmonary tissues on day 14 from representative samples in each group administered with PBS, BLM, and ICAM-1 Ab, according to Experimental Example 4.4. Here, "PBS #1" to "PBS #3" refer to individual 1 to 3 of a group administered with PBS only, "BLM #1" to "BLM #3" refer to individual 1 to 3 of a group administered with bleomycin only, and "BLM + ICAM-1 Ab #1" to "BLM + ICAM-1 Ab #3" refer to individual 1 to 3 of a group administered with ICAM-1 antibody after bleomycin-induced fibrosis developed.

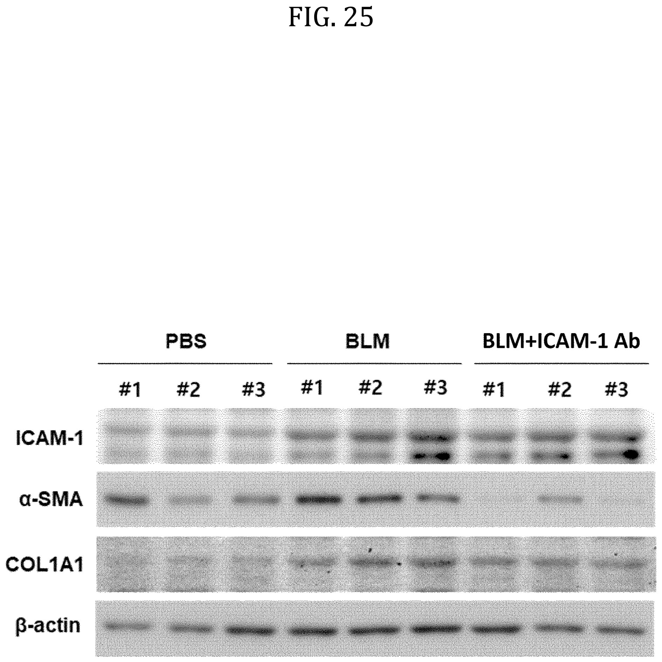

FIG. 25 shows results of analysis of expression levels of α-SMA, Col1A1, and ICAM-1 proteins in pulmonary tissues on day 14 from representative samples in each group administered with PBS, BLM, and ICAM-1 Ab, according to Experimental Example 4.5. Here, "PBS" refers to a group administered with PBS only, "BLM" refers to a group administered with bleomycin only, and "BLM + ICAM-1 Ab" refers to a group administered with bleomycin followed by an ICAM-1 antibody. The darker band, the higher level of protein expression.

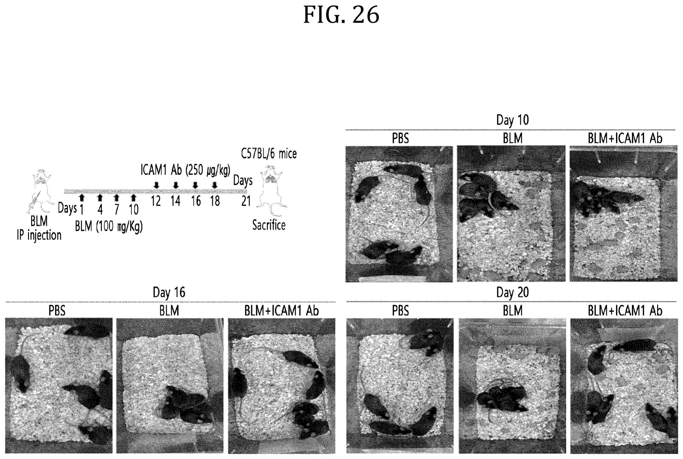

FIG. 26 shows results of behavioral observations of mice in each group administered with PBS, BLM, and ICAM-1 Ab, according to Experimental Example 4.6. A left upper panel schematically illustrates an administration schedule of bleomycin (BLM) and ICAM-1 neutralizing antibody (ICAM-1 Ab) to mice. "Day 10", "Day 16", and "Day 20" indicate results of behavioral observations of mice in each group conducted on experimental days 10, 16, and 20, respectively. Here, "PBS" refers to a group administered with PBS only, "BLM" refers to a group administered with bleomycin only, and "BLM + ICAM-1 Ab" refers to a group administered with bleomycin followed by an ICAM-1 antibody.

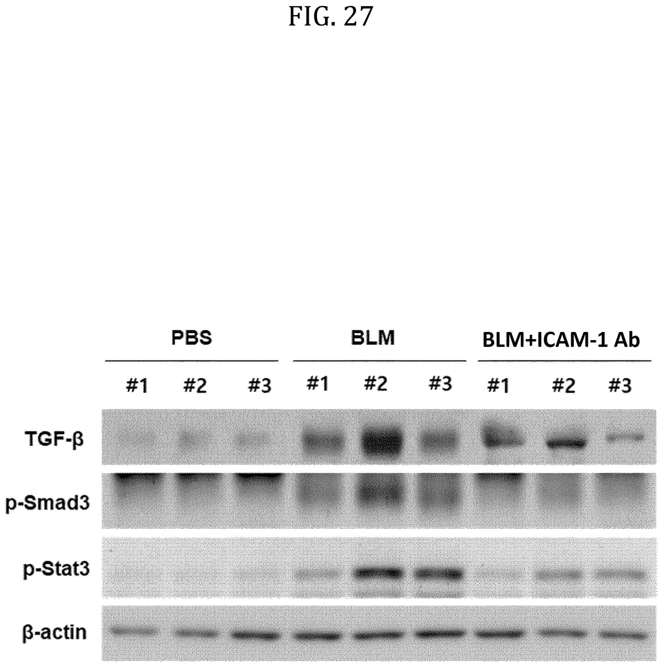

FIG. 27 shows results of Western blot analysis performed in pulmonary tissues collected from individual animals (three in each group, #1 to #3) in each group, according to Experimental Example 5.1, to assess expression level of TGF-β protein, and whether Smad3 and Stat3 are activated or not. Here, "PBS" refers to a group administered with PBS only, "BLM" refers to a group administered with bleomycin only, and "BLM + ICAM-1 Ab" refers to a group administered with bleomycin followed by an ICAM-1 antibody.

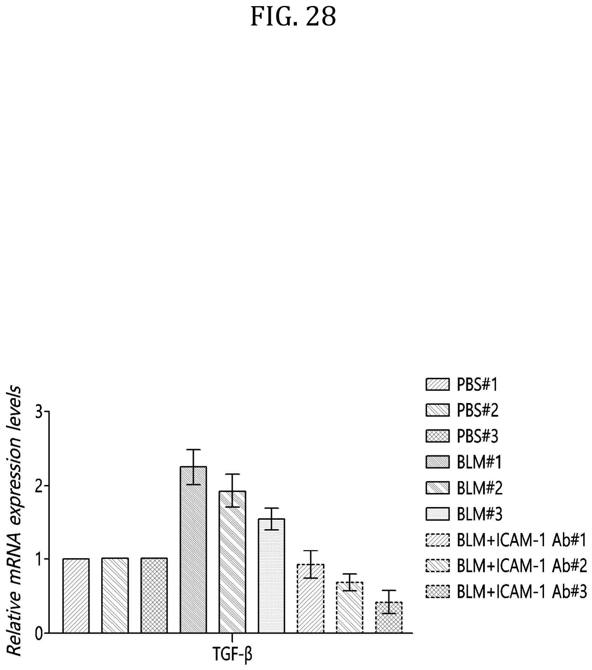

FIG. 28 shows results of measuring expression level of TGF-β gene using q-PCR in pulmonary tissues collected from individual animals (#1 to #3) in each group, according to Experimental Example 5.2. Here, "PBS #1" to "PBS #3" refer to individual 1 to 3 of a group administered with PBS only, "BLM #1" to "BLM #3" refer to individual 1 to 3 of a group administered with bleomycin only, and "BLM + ICAM-1 Ab #1" to "BLM + ICAM-1 Ab #3" refer to individual 1 to 3 of a group administered with bleomycin followed by ICAM-1 antibody.

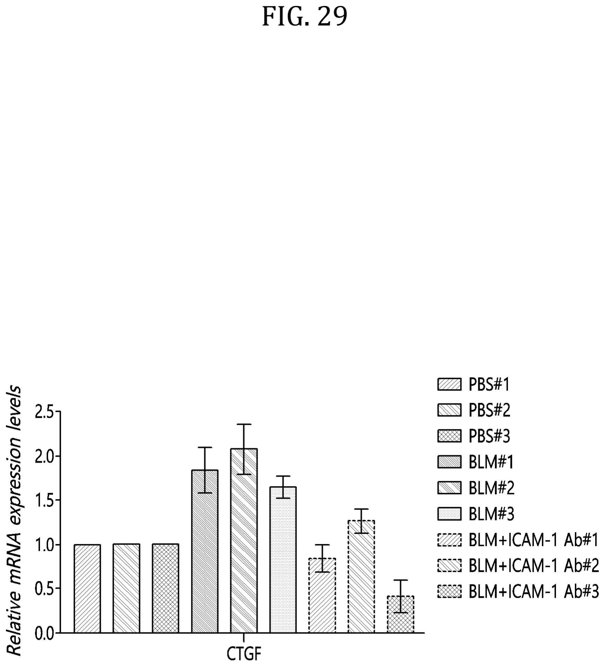

FIG. 29 shows results of measuring expression level of CTGF gene using q-PCR in pulmonary tissues collected from individual animals (#1 to #3) in each group, according to Experimental Example 5.2. Here, "PBS #1" to "PBS #3" refer to individual 1 to 3 of a group administered with PBS only, "BLM #1" to "BLM #3" refer to individual 1 to 3 of a group administered with bleomycin only, and "BLM + ICAM-1 Ab #1" to "BLM + ICAM-1 Ab #3" refer to individual 1 to 3 of a group administered with ICAM-1 antibody.

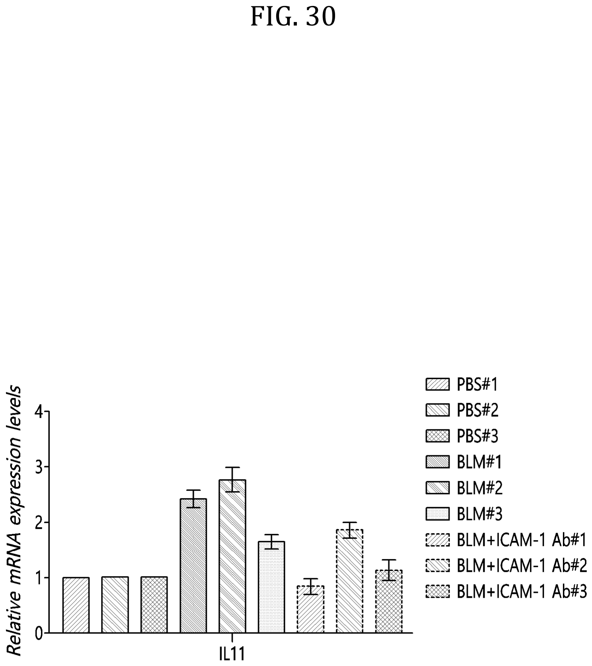

FIG. 30 shows results of measuring expression level of IL11 gene using q-PCR in pulmonary tissues collected from individual animals (#1 to #3) in each group, according to Experimental Example 5.2. Here, "PBS #1" to "PBS #3" refer to individual 1 to 3 of a group administered with PBS only, "BLM #1" to "BLM #3" refer to individual 1 to 3 of a group administered with bleomycin only, and "BLM + ICAM-1 Ab #1" to "BLM + ICAM-1 Ab #3" refer to individual 1 to 3 of a group administered with bleomycin followed by ICAM-1 antibody.

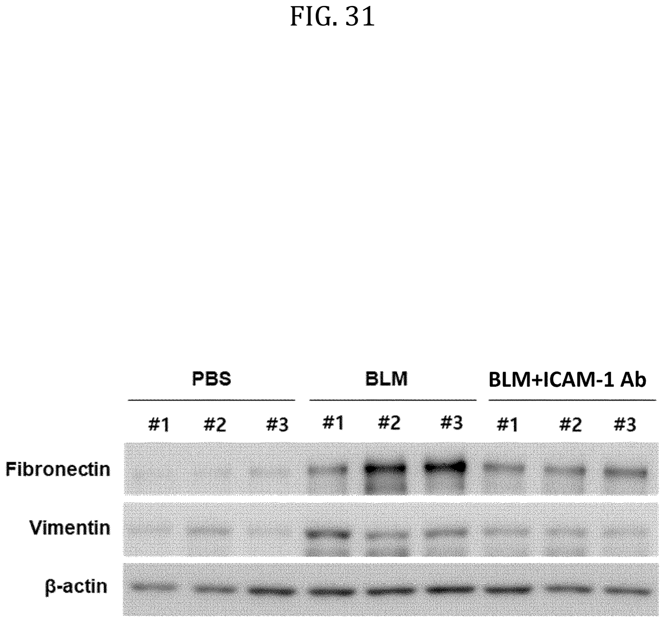

FIG. 31 shows results of analysis of expression levels of Fibronectin and Vimentin proteins in pulmonary tissues collected from individual animals (#1 to #3) in each group, according to Experimental Example 5.3. Here, "PBS #1" to "PBS #3" refer to individual 1 to 3 of a group administered with PBS only, "BLM #1" to "BLM #3" refer to individual 1 to 3 of a group administered with bleomycin only, and "BLM + ICAM-1 Ab #1" to "BLM + ICAM-1 Ab #3" refer to individual 1 to 3 of a group administered with bleomycin followed by ICAM-1 antibody.

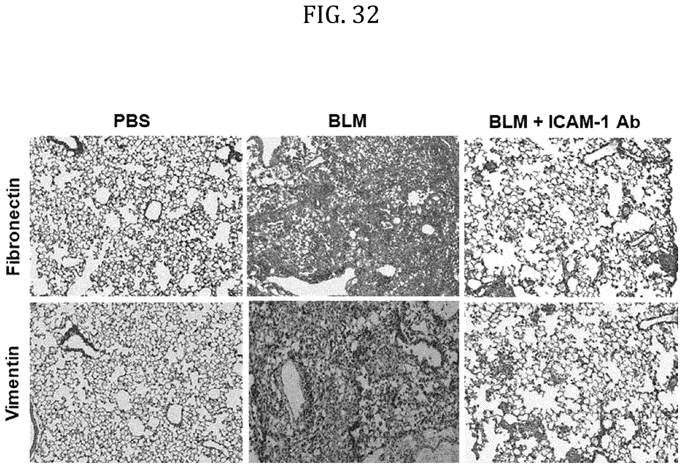

FIG. 32 shows results of immunohistochemical staining for Fibronectin and Vimentin proteins in pulmonary tissues from representative samples in each group, according to Experimental Example 5.3, wherein these Fibronectin and Vimentin proteins were stained brown to observe their accumulation, and stained proteins were observed under a microscope. Here, "PBS" refers to a group administered with PBS only, "BLM" refers to a group administered with bleomycin only, and "BLM + ICAM-1 Ab" refers to a group administered with bleomycin followed by an ICAM-1 antibody.

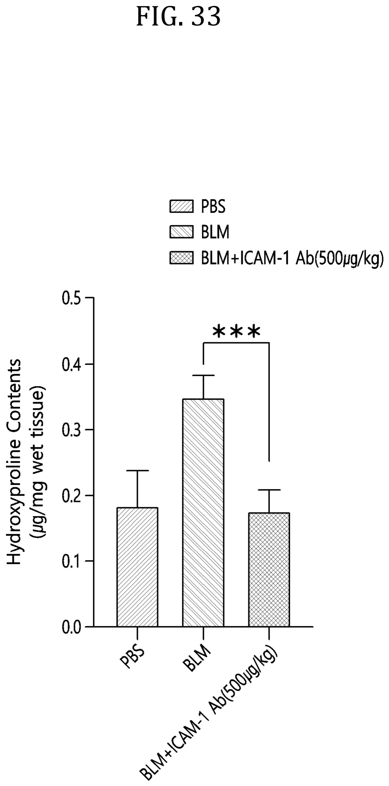

FIG. 33 shows results of analyzing changes in hydroxyproline content of pulmonary tissue samples on day 14 collected from each group, according to Experimental Example 5.4. Here, "PBS" refers to a group administered with PBS only, "BLM" refers to a group administered with bleomycin only, and "BLM + ICAM-1 Ab" refers to a group administered with bleomycin followed by an ICAM-1 antibody.

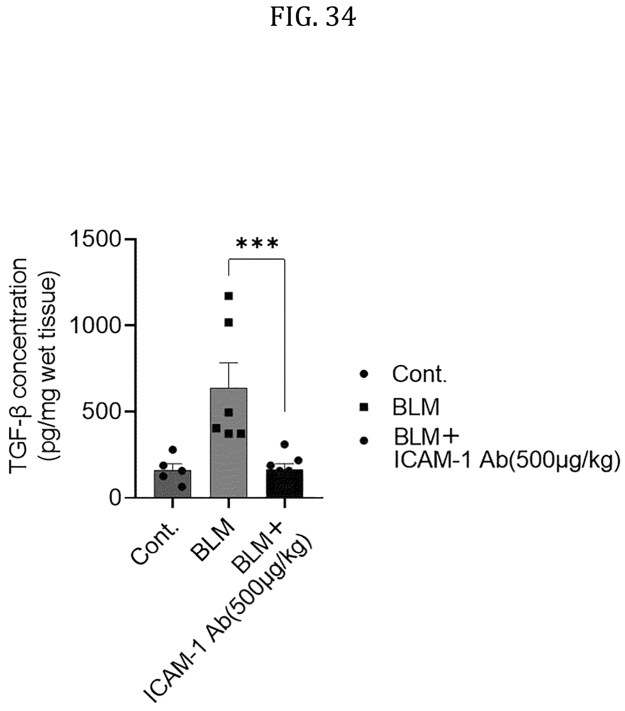

FIG. 34 shows results of analyzing change in TGF-β of pulmonary tissue samples on day 14 collected from each group, according to Experimental Example 5.5. Here, "Cont" refers to a group administered with PBS only, "BLM" refers to a group administered with bleomycin only, and "BLM + ICAM-1 Ab" refers to a group administered with bleomycin followed by an ICAM-1 antibody.

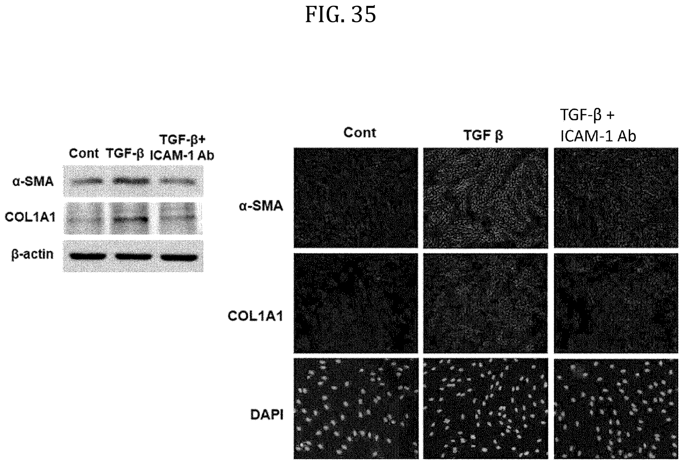

FIG. 35 shows results of Experiment 6.1, in which skin fibroblasts were administered with TGF-β for 48 hours to induce skin fibrosis, and changes in expression levels of fibrosis markers α-SMA and Col1A1 at both protein and gene levels were observed. A left panel shows results of analyzing expression levels of α-SMA and Col1A1 proteins using Western blotting, while a right panel shows fluorescence staining results: α-SMA was stained green, and Col1A1 was stained red to visualize expression levels of these proteins. Here, "Cont" refers to a group administered with PBS only, "TGF-β" refers to a group administered with TGF-β only, and "TGF-β + ICAM-1 Ab" refers to a group co-administered with TGF-β and ICAM-1 antibody.

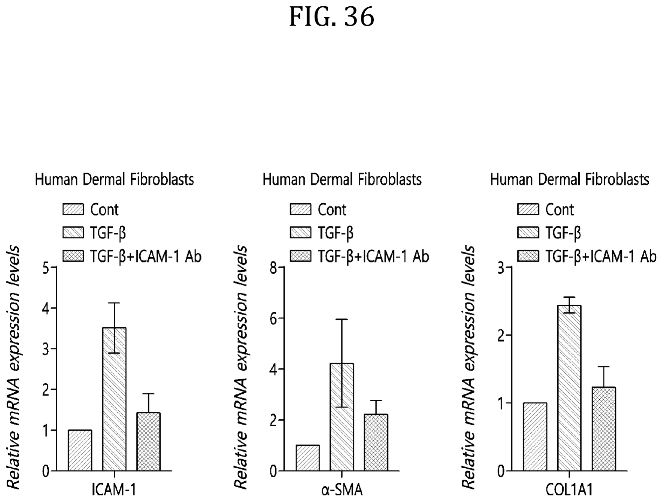

FIG. 36 shows results of q-PCR analysis of expression levels of ICAM-1, α-SMA, and Col1A1 genes in each group, according to Experimental Example 6.2. Here, "Cont" refers to a group in which skin fibroblasts were administered with PBS only, "TGF-β" refers to a group in which skin fibroblasts were administered with TGF-β only, and "TGF-β + ICAM-1 Ab" refers to a group in which skin fibroblasts were co-administered with TGF-β and an ICAM-1 antibody.

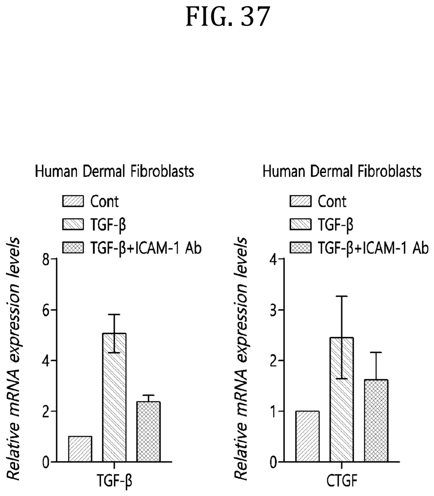

FIG. 37 shows results of q-PCR analysis of expression levels of TGF-β and CTGF genes in each group, according to Experimental Example 6.2. Here, "Cont" refers to a group in which skin fibroblasts was administered with PBS only, "TGF-β" refers to a group a group in which skin fibroblasts was administered with TGF-β only, and "TGF-β + ICAM-1 Ab" refers to a group in which skin fibroblasts was co-administered with both TGF-β and an ICAM-1 antibody.

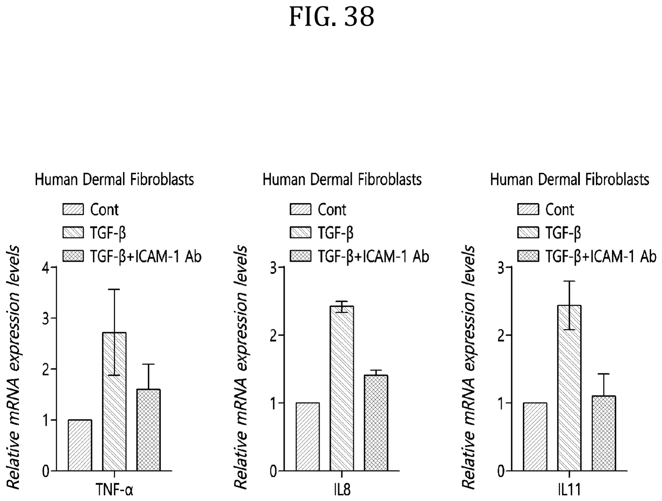

FIG. 38 shows results of q-PCR analysis of expression levels of TNF-α, IL-8, and IL-11 genes in each group, according to Experimental Example 6.1. Here, "Cont" refers to a group in which skin fibroblasts were administered with only PBS, "TGF-β" refers to a group in which skin fibroblasts were administered with TGF-β only, and "TGF-β + ICAM-1 Ab" refers to a group in which skin fibroblasts were co-administered with both TGF-β and an ICAM-1 antibody.

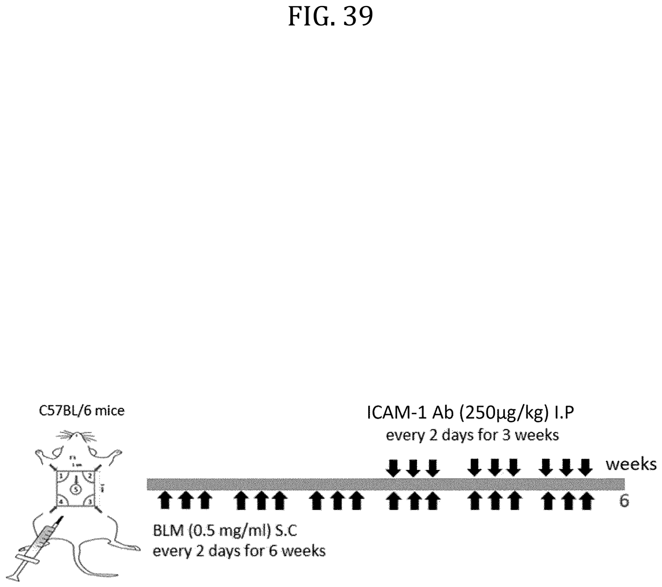

FIG. 39 schematically illustrates an administration schedule of bleomycin (BLM) and ICAM-1 neutralizing antibody (ICAM-1 Ab) in an animal experimental model, according to Experimental Example 7.1.

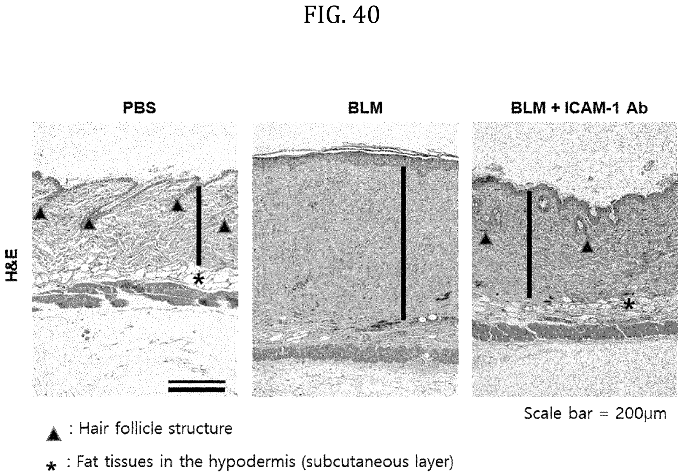

FIG. 40 shows microscopic analysis results of mouse skin tissues from each group following H&E staining in order to find morphology of tissues, according to Experimental Example 7.2. Here, "PBS" refers to a control group administered with PBS only, "BLM" refers to an experimental group administered with bleomycin only, and "BLM + ICAM-1 Ab" refers to a group co-administered with both bleomycin and ICAM-1 antibody.

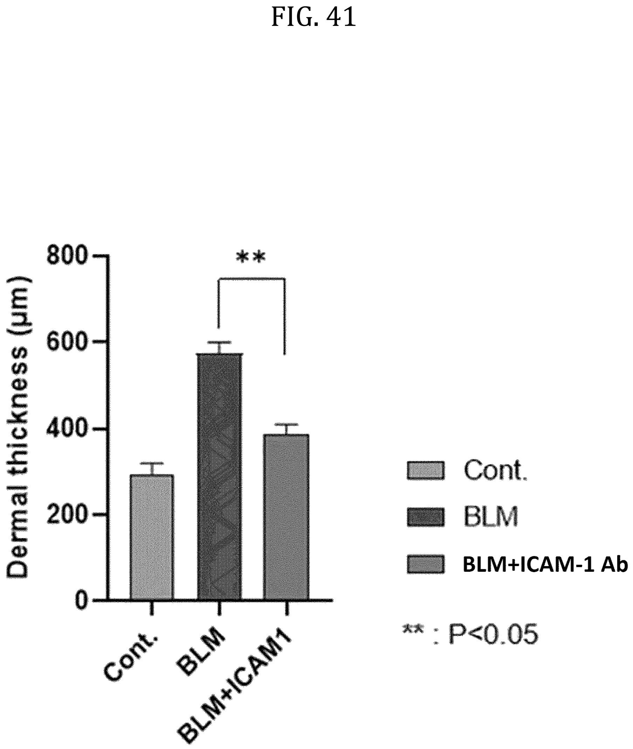

FIG. 41 shows microscopic analysis results of mouse skin tissues from each group following H&E staining in order to find morphology of tissues, and measure thickness of mice skin tissues, according to Experimental Example 7.2. Here, "Cont" refers to a control group administered with PBS only, "BLM" refers to an experimental group administered with bleomycin only, and "BLM + ICAM-1 Ab" refers to a group co-administered with both bleomycin and ICAM-1 antibody.

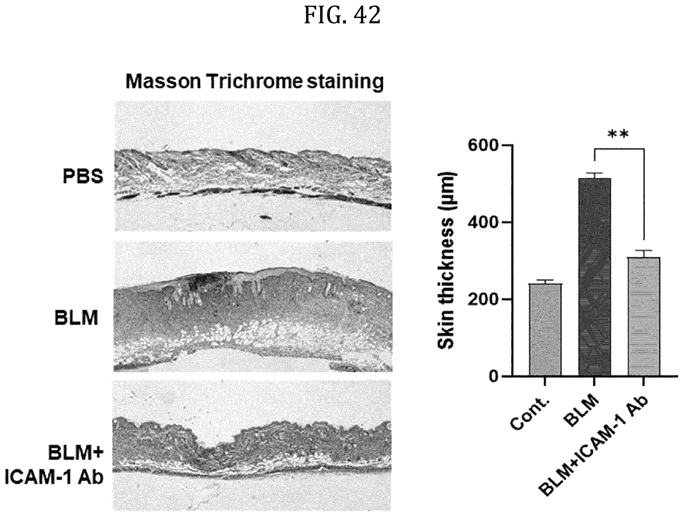

FIG. 42 shows microscopic analysis results of collagen present in mouse skin tissues from each group, in which Masson's trichrome staining was performed, according to Experimental Example 7.2. Here, "Cont" refers to a control group administered with PBS only, "BLM" refers to an experimental group administered with bleomycin only, and "BLM + ICAM-1 Ab" refers to a group co-administered with both bleomycin and ICAM-1 antibody.

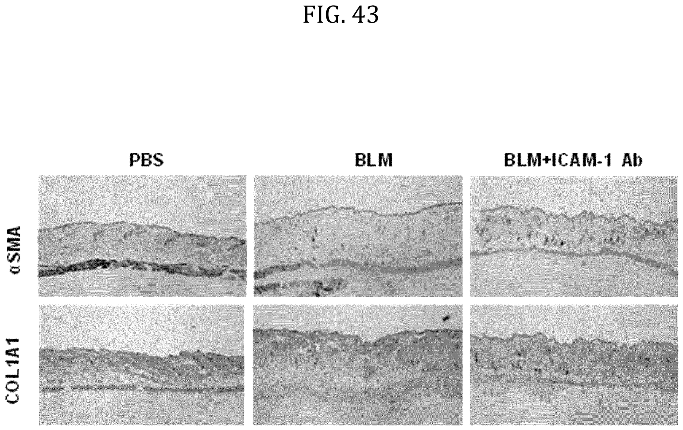

FIG. 43 shows microscopic analysis results of α-SMA and Col1A1 proteins in mouse skin tissues from each group, in which these α-SMA and Col1A1 proteins were visualized by immunohistochemical (IHC) staining in brown color, according to Experimental Example 7.2. Here, "PBS" refers to a control group administered with PBS only, "BLM" refers to an experimental group administered with bleomycin only, and "BLM + ICAM-1 Ab" refers to a group co-administered with both bleomycin and ICAM-1 antibody.

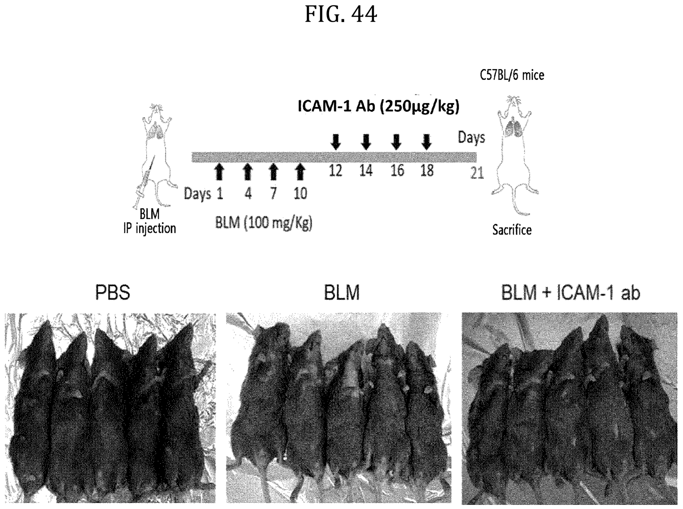

FIG. 44 shows results of an animal model experiment for BLM-induced skin fibrosis performed, according to Experimental Example 7.3. Upper panel schematically illustrates administration schedule of BLM (bleomycin) and ICAM-1 (ICAM-1 neutralizing antibody) to mice. Lower panel shows experimental results of an animal model from each treatment group. Here, "PBS" refers to a control group administered with PBS only, "BLM" refers to an experimental group administered with bleomycin only, and "BLM + ICAM-1 Ab" refers to a group co-administered with both bleomycin and ICAM-1 antibody.

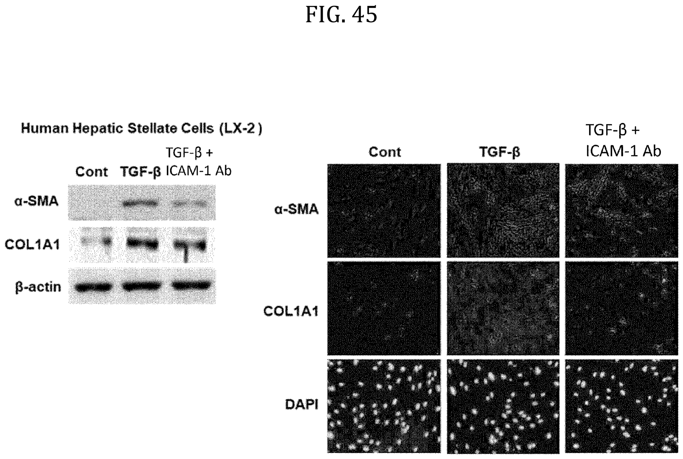

FIG. 45 shows results of expression analysis of α-SMA and Col1A1 proteins in each group, according to Experimental Example 8.1. Left panel shows results of Western blot analysis for α-SMA and Col1A1 protein expression. Right panel shows results of immunofluorescence staining, in which α-SMA is visualized in green, and Col1A1 in red. Here, "Cont" refers to a control group administered with PBS only, "TGF-β" refers to an experimental group administered with TGF-β only, and "TGF-β + ICAM-1 Ab" refers to a group co-administered with both bleomycin and ICAM-1 neutralizing antibody.

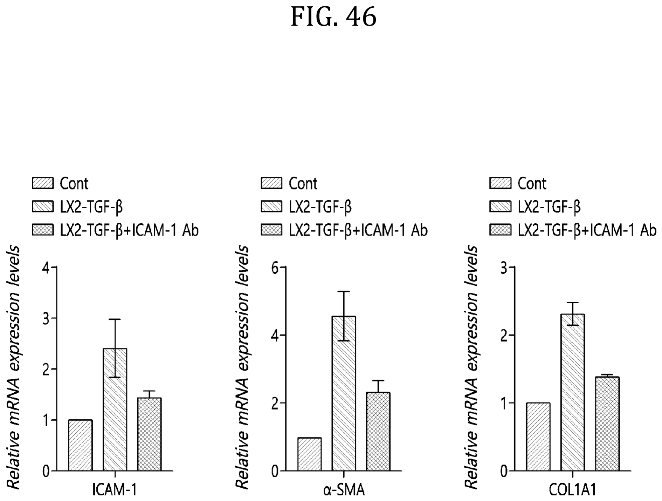

FIG. 46 shows results of expression analysis of α-SMA, Col1A1, and ICAM-1 genes in cell samples collected from each group, using q-PCR, according to Experimental Example 8.1. Here, "Cont" refers to a control group administered with PBS only, "LX2-TGF-β" refers to an experimental group administered with TGF-β only, and "LX2-TGF-β + ICAM-1 Ab" refers to a group co-administered with both TGF-β and ICAM-1 neutralizing antibody.

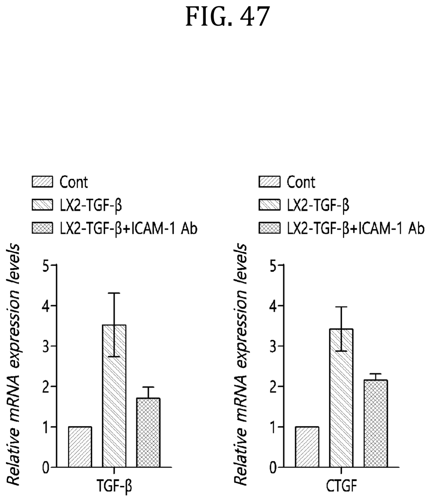

FIG. 47 shows results of expression analysis of TGF-β, and CTGF genes in cell samples collected from each group, using q-PCR, according to Experimental Example 8.1. Here, "Cont" refers to a control group administered with PBS only, "LX2-TGF-β" refers to an experimental group administered with TGF-β only, and "LX2-TGF-β + ICAM-1 Ab" refers to a group co-administered with both TGF-β and ICAM-1 neutralizing antibody.

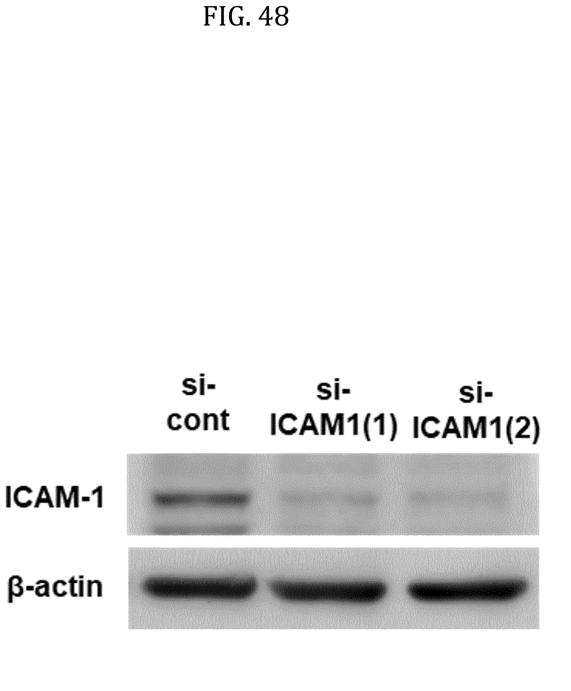

FIG. 48 shows results of Western blot analysis performed to confirm whether when administration of 100 nM ICAM-1-targeting siRNA in normal pulmonary fibroblasts is performed, ICAM-1 protein expression is also suppressed or not. Here, "si-cont" refers to a control group administered with random RNA, "si-ICAM1 (1)" refers to an experimental group administered with a siRNA of SEQ ID NO: 4, and "si-ICAM1 (2)" refers to a group administered with a siRNA of SEQ ID NO: 6.

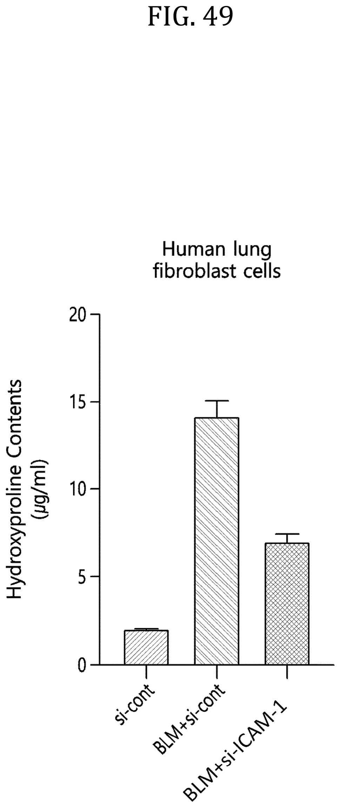

FIG. 49 shows results of hydroxyproline content measurement for each group, according to Experimental Example 10.1. Here, "si-cont" refers to a control group administered with a siRNA with random sequences; "BLM + si-cont" refers to an experimental group administered with both bleomycin and siRNA with random sequences; and "BLM + si-ICAM1" refers to an experimental group administered with both bleomycin and an ICAM-1-targeting siRNA.

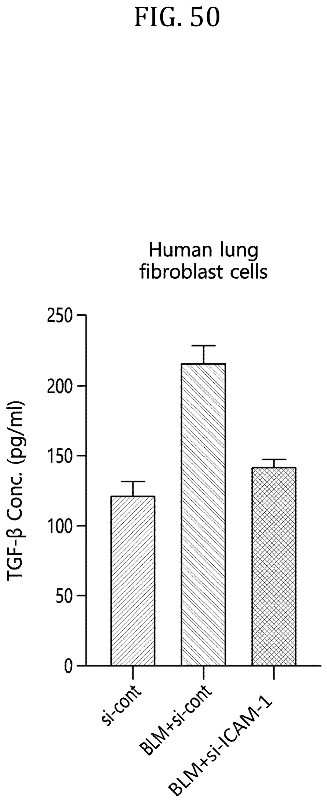

FIG. 50 shows results of TGF-β concentration measurements for each group, according to Experimental Example 10.1. Here, "si-cont" refers to a control group administered with a siRNA having any sequences; "BLM + si-cont" refers to an experimental group administered with both bleomycin and a siRNA with random sequences; and "BLM + si-ICAM1" refers to an experimental group administered with both bleomycin and an ICAM-1-targeting siRNA.

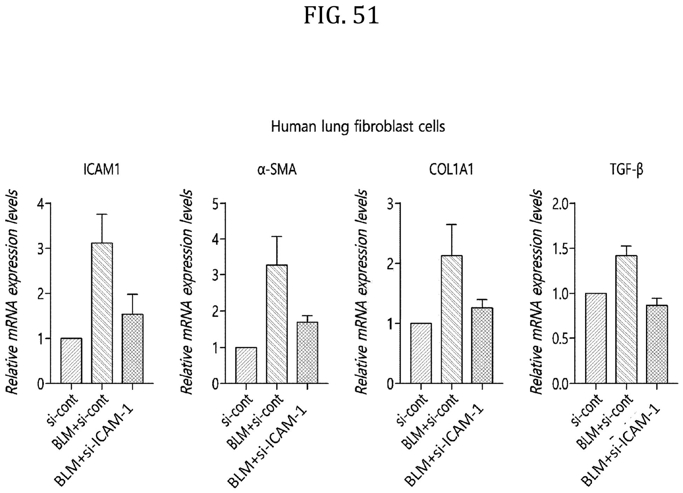

FIG. 51 shows results of expression level measurements of ICAM-1, α-SMA, CollA1, and TGF-β mRNA in each group, according to Experimental Example 10.1. Here, "si-cont" refers to a control group administered with a siRNA having any sequences; "BLM + si-cont" refers to an experimental group administered with both bleomycin and a siRNA with random sequences; and "BLM + si-ICAM1" refers to an experimental group administered with both bleomycin and an ICAM-1-targeting siRNA.

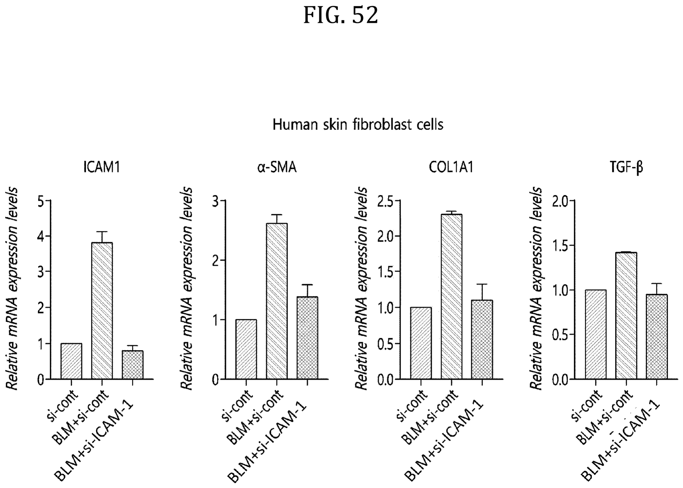

FIG. 52 shows results of expression level measurements of ICAM-1, α-SMA, CollA1, and TGF-β mRNA for each group, according to Experimental Example 10.2. Here, "si-cont" refers to a control group administered with a siRNA with random sequences; "BLM + si-cont" refers to an experimental group administered with both bleomycin and siRNA with random sequences; and "BLM + si-ICAM1" refers to an experimental group administered with both bleomycin and an ICAM-1-targeting siRNA.

### Best Mode

The following describes exemplary embodiments regarded as the best mode for carrying out the present disclosure. These embodiments include certain implementations of the present disclosure disclosed in the present specification, but not all possible embodiments. The embodiments described in this section are merely illustrative, and should not be construed as the exclusive "best mode" of the invention. Those skilled in the art will appreciate that various modifications and more preferable implementations may be conceived based on the embodiments described herein, and such variations are also to be considered as part of the best mode for carrying out the invention.

The present disclosure provides an ICAM-1 neutralizing antibody for use in treating fibrosis.

In one embodiment, the ICAM-1 neutralizing antibody may neutralize ICAM-1 having an amino acid sequence as below:

MAPSSPRPALPALLVLLGALFPGPGNAQTSVSPSKV
ILPRGGSVLVTCSTSCDQPKLLGIETPLPKKELLLPGNNR
KVYELSNVQEDSQPMCYSNCPDGQSTAKTFLTVYWTPERV
ELAPLPSWQPVGKNLTLRCQVEGGAPRANLTVVLLRGEKE
LKREPAVGEPAEVTTTVLVRRDHHGANFSCRTELDLRPQG
LELFENTSAPYQLQTFVLPATPPQLVSPRVLEVDTQGTVV
CSLDGLFPVSEAQVHLALGDQRLNPTVTYGNDSFSAKASV
SVTAEDEGTQRLTCAVILGNQSQETLQTVTIYSFPAPNVI
LTKPEVSEGTEVTVKCEAHPRAKVTLNGVPAQPLGPRAQL
LLKATPEDNGRSFSCSATLEVAGQLIHKNQTRELRVLYGP
RLDERDCPGNWTWPENSQQTPMCQAWGNPLPELKCLKDGT
FPLPIGESVTVTRDLEGTYLCRARSTQGEVTRKVTVNVLS
PRYEIVIITVVAAAVIMGTAGLSTYLYNRQRKIKKYRLQQ
AQKGTPMKPNTQATPP (SEQ ID NO:1)

In one embodiment, an epitope of the ICAM-1 neutralizing antibody may have an amino acid sequence as below:

QTSVSPSKVILPRGGSVLVTCSTSCDQPKLLGIETPLPKKELLLPGNNRK
VYELSNVQEDSQPMCYSNCPDGQSTAKTFLTVYWTPERVE
LAPLPSWQPVGKNLTLRCQVEGGAPRANLTVVLLRGEKEL
KREPAVGEPAEVTTTVLVRRDHHGANFSCRTELDLRPQGL
ELFENTSAPYQLQTFVLPATPPQLVSPRVLEVDTQGTVVC
SLDGLFPVSEAQVHLALGDQRLNPTVTYGNDSFSAKASVS
VTAEDEGTQRLTCAVILGNQSQETLQTVTIYSFPAPNVIL
TKPEVSEGTEVTVKCEAHPRAKVTLNGVPAQPLGPRAQLL
LKATPEDNGRSFSCSATLEVAGQLIHKNQTRELRVLYGPR
LDERDCPGNWTWPENSQQTPMCQAWGNPLPELKCLKDGTF
PLPIGESVTVTRDLEGTYLCRARSTQGEVTRKVTVNVLSPRYE (SEQ ID NO:2)

In one embodiment, the fibrosis may be selected from a group consisting of:

hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; chronic pancreatitis; duct obstruction; autoimmune-interstitial pulmonary disease such as ankylosing spondylitis, and rheumatoid arthritis; connective tissue disease-interstitial pulmonary disease such as rheumatoid arthritis-interstitial pulmonary disease; and non-idiopathic pulmonary fibrosis-interstitial pulmonary disease.

In one embodiment, the ICAM-1 neutralizing antibody may cause to degrade extracellular matrix accumulated in fibrotic tissues.

In one embodiment, the extracellular matrix accumulated in fibrotic tissues may be collagen.

In one embodiment, the ICAM-1 neutralizing antibody may inactivate myofibroblasts in fibrotic tissues.

In one embodiment, inactivating myofibroblasts in fibrotic tissues means at least one selected from the following:
(a) degrading the extracellular matrix secreted by the myofibroblasts;
(b) inhibiting secretion of the extracellular matrix by the myofibroblasts; and
(c) reducing myofibroblasts in fibrotic tissues.

In one embodiment, the extracellular matrix of (a) and (b) may be collagen.

In one embodiment, (b) may be inducing reduction in expression level of a COL1A1 gene in fibrotic tissues.

In one embodiment, (c) may be inducing reduction in expression level of a COL1A1 gene in fibrotic tissues.

In one embodiment, (c) may occur as a result of reverting the myofibroblasts to epithelial cells.

In one embodiment, the ICAM-1 neutralizing antibody may inhibit ICAM-1-mediated signaling in fibrotic tissues.

In one embodiment, the ICAM-1-mediated signaling may be at least one selected from a group consisting of:
(a) epithelial-to-mesenchymal transition (EMT); and
(b) extracellular matrix remodeling in tissues.

The present disclosure provides a method of preventing or treating fibrosis, the method comprising:
administering a therapeutically effective amount of the ICAM-1 neutralizing antibody of any one of the above embodiments to a subject.

The present disclosure provides a pharmaceutical composition for treating fibrosis, the composition comprising:
a therapeutically effective amount of the ICAM-1 neutralizing antibody of any one of the above embodiments; and
pharmaceutically acceptable carriers.

In one embodiment, the pharmaceutically acceptable carrier may be at least one selected from a group consisting of:
binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol (PEG) wax; sweeteners; flavoring agents; syrups; liquid carriers such as fatty oils; sterile aqueous solutions; propylene glycol; polyethylene glycol; injectable esters such as ethyl oleate; suspending agents; emulsifiers; lyophilized preparations; topical formulations; stabilizers; buffers; animal oils; vegetable oils; waxes; paraffin; starches; tragacanth; cellulose derivatives; polyethylene glycol; silicone; bentonite; silica; talc; zinc oxide; and appropriate combinations thereof.

The present disclosure provides use of the ICAM-1 neutralizing antibody of any one of the above embodiments for preparing a fibrosis treatment.

### Mode for Disclosure

### Definition of terms

### About

As used herein, the term "about" refers to a variation of 30%, 25%, 20%, 15%, 10%, 9%, 8%, 7%, 6%, 50%, 4%, 3%, 2%, 1%, or 0% with respect to a referenced amount, level, value, number, frequency, percentage, dimension, size, quantity, weight, or length.

### Description of amino acid sequence

Unless otherwise stated, amino acid sequences in the present specification are described using either one-letter or three-letter abbreviation system, in a direction from N-terminus to C-terminus. For example, "RNVP" refers to a peptide in which arginine (R), asparagine (N), valine (V), and proline (P) are sequentially linked from N-terminus to C-terminus. In another example, "Thr-Leu-Lys" refers to a peptide in which threonine, leucine, and lysine are sequentially linked from N-terminus to C-terminus. For amino acids that cannot be represented using the one-letter abbreviation, other characters may be used, and supplemented with additional explanation.

According to the one-letter or three-letter abbreviation system, a notation for each amino acid used herein is as follows: alanine (Ala, A); arginine (Arg, R); asparagine (Asn, N); aspartic acid (Asp, D); cysteine (Cys, C); glutamic acid (Glu, E); glutamine (Gln, Q); glycine (Gly, G); histidine (His, H); isoleucine (Ile, I); leucine (Leu, L); lysine (Lys, K); methionine (Met, M); phenylalanine (Phe, F); proline (Pro, P); serine (Ser, S); threonine (Thr, T); tryptophan (Trp, W); tyrosine (Tyr, Y); and valine (Val, V).

### Treating or treatment

As used herein, the term "treating" refers to any direct or indirect action or measure that results in elimination, alleviation, mitigation, suppression, improvement, or prevention of diseases, disorders, conditions, and/or symptoms in a subject. As used herein, the term "treatment" refers to any substance (e.g., a compound or peptide) capable of producing the aforementioned therapeutic effect when appropriately administered to a subject. Furthermore, the terms "treating" and "treatment" also encompass all meanings as understood by those skilled in the art.

### Pathogenesis of fibrosis

### Overview

Fibrosis is a pathological condition characterized by an excessive accumulation of extracellular matrix, particularly collagen, in tissues. When fibrosis develops, fibrotic tissues lose their original function, and become rigid due to accumulation of extracellular matrix. It is known that fibrosis causes irreversible and permanent damage to tissues, and its progression can lead to dysfunction of fibrotic tissues, and ultimately result in patient mortality. Fibrosis may arise from persistent stimulation or severe injury to tissues, and is also a common and final pathological outcome of many chronic inflammatory diseases.

Pathologically, it can be understood that fibrosis develops as a result of an abnormal activation of tissue repair mechanisms. Regardless of an initial cause, or tissues in which fibrosis develops, activated myofibroblasts may be observed in all fibrotic tissues. These myofibroblasts are known to be a main cause of extracellular matrix accumulation in tissues. The myofibroblasts are cells that appear during activation of wound healing processes following tissue injury, and actively secrete extracellular matrix, particularly fibrillar connective tissue such as collagen, thereby facilitating tissue repair. However, when myofibroblasts become aberrantly activated due to unknown reason, and this activation is sustained, extracellular matrix produced by these myofibroblasts continues to accumulate in tissues, ultimately leading to fibrotic tissues.

### Fibrotic tissues

Fibrosis can develop in nearly all tissues of a body, and it is known that fibrosis in various tissues can lead to a wide range of diseases, disorders, and/or pathological conditions. For example, fibrosis has been reported to develop in tissues such as skin, brain, nervous system, heart, bone marrow, liver, gut, joints, eyes, lungs, kidneys, retroperitoneum, mediastinum, and/or pancreas.

### Tissue injury and activation of myofibroblasts

Epithelial cells in tissues may be injured or stimulated by various factors, including bacterial infection, toxic substances, drugs, stress, inflammation, or trauma. When tissue is injured or stimulated, a variety of signaling substances such as cytokines are secreted in tissues. Numerous signaling substances secreted and exchanged by epithelial cells and many kind of immune cells induce immune and inflammatory responses on one hand, and on the other hand, cause activation of residential fibroblasts and myofibroblasts. Specifically, residential fibroblasts in tissues become activated, and differentiate into myofibroblasts which secrete extracellular matrix (ECM) necessary for tissue repair. Meanwhile, epithelial-to-mesenchymal transition (EMT) of tissue epithelial cells is induced, and mesenchymal stem cells further differentiate into myofibroblasts, followed by being activated. These activated myofibroblasts actively secrete extracellular matrix, particularly fibrillar connective tissue such as collagen.

### Maintenance of myofibroblasts activation

Under normal conditions, immune and inflammatory responses would eliminate stimuli, or causes of cellular injure, and following cellular tissue repair performed by extracellular matrix secreted by activated myofibroblasts, and the immune response, inflammatory response, and myofibroblast activation are suppressed, allowing tissues to return to a normal state. However, when tissues are subjected to persistent stress or stimulation such that immune and inflammatory responses remain continuously active, or when myofibroblast activation is sustained due to any reasons, myofibroblasts remain in an activated state, and continuously produce and secrete extracellular matrix.

### Progress of fibrosis

When activation of myofibroblasts is sustained, extracellular matrix such as collagen continues to accumulate in tissues. This persistent accumulation disrupts a normal architecture of normal tissues, impairs functions of normal tissues, and ultimately exerts a fatal impact on patient survival.

### Limitations of conventional fibrosis treatment

Accumulation of extracellular matrix due to fibrosis, or fibrosis has been considered irreversible. Moreover, since too many factors are involved in onset and progression of fibrosis, definitive causal factors have not been clearly identified to date. As a result, fibrosis has been reported to develop in various tissues, and has also been reported as a pathological outcome of numerous chronic inflammatory diseases; however, effective methods of treating fibrosis, or treatments for treating fibrosis remain unavailable.

In addition, treatments for treating fibrosis, which have been studied and disclosed to date, have just demonstrated efficacy in partially removing extracellular matrix that has already accumulated, rather than directly targeting underlying cause of fibrosis, myofibroblasts. However, extracellular matrix will continue to be secreted and accumulated in tissues, unless secretion of extracellular matrix by myofibroblasts is inhibited, inducing epithelial-to-mesenchymal transition (EMT) of epithelial cells of tissues is suppressed, and/or activation of residential fibroblasts and their differentiation into myofibroblasts are inhibited to reduce or eliminate myofibroblasts themselves. Therefore, an effect of the fibrosis treatments that merely exhibit partial extracellular matrix removal, remains limited.

### Fibrosis treatment containing ICAM-1 inhibitor

### Overview of fibrosis treatment

The present specification discloses a fibrosis treatment containing the ICAM-1 inhibitor. The ICAM-1 inhibitor functions to directly and/or indirectly block or interfere with exerting functions of ICAM-1 in vivo. Specifically, the ICAM-1 inhibitor may inhibit interaction between ICAM-1 proteins and other molecules, or suppress expression level of the ICAM-1 protein. The present inventors have discovered that inhibition of ICAM-1 function in fibrotic tissues results in reversion of the fibrotic tissues to normal tissues, and treatment of fibrosis, and have elucidated an underlying therapeutic mechanism through research. The fibrosis treatment containing the ICAM-1 inhibitor can inhibit ICAM-1-mediated signaling, and as a result, suppress epithelial-to-mesenchymal transition (EMT) of tissue epithelial cells, and/or accumulation of extracellular matrix, which are/is associated with ICAM-1-mediated signaling. The fibrosis treatment containing the ICAM-1 inhibitor has its therapeutic mechanism performed by inhibiting induction of epithelial-to-mesenchymal transition (EMT) in epithelial cells of tissue, preventing differentiation into myofibroblasts through suppression of residential fibroblast activation, and/or directly acting on myofibroblasts to reduce myofibroblasts.

The present inventors have discovered that inhibition of ICAM-1 in fibrotic tissues exerts a direct therapeutic effect on fibrosis, namely, degradation of accumulated extracellular matrix such as collagen, and reduction of myofibroblasts, thereby reverting fibrotic tissues to normal tissues. Furthermore, through additional experiments, the present inventors have elucidated a therapeutic mechanism of the ICAM-1 inhibitor in treatment of fibrosis.

### ICAM-1

Intercellular adhesion molecule-1 (ICAM-1), also referred to as Cluster of Differentiation 54 (CD54), is a protein belonging to an immunoglobulin superfamily, which includes antibodies and T-cell receptors, etc. It is known that ICAM-1 is a transmembrane protein predominantly expressed on leukocytes and vascular endothelial cells, which functions as a cell adhesion molecule. It is also known that ICAM-1 may bind to LFA-1, a receptor expressed on leukocytes. ICAM-1 is activated in response to secretion of cytokines such as interleukin-1 (IL-1) and tumor necrosis factor (TNF). When activated on vascular endothelium, ICAM-1 binds to LFA-1 on leukocytes, capturing and transporting leukocytes into tissues. In addition, it is revealed that ICAM-1 is also involved in immune responses, and partially participates in intercellular signaling.

### Meaning of ICAM-1 inhibitor

As used herein, the term "ICAM-1 inhibitor" refers to a substance that ultimately inhibits function of ICAM-1. The ICAM-1 inhibitor is not particularly limited in type, shape, or composition, so long as it is capable of inhibiting function of ICAM-1. Specifically, inhibition of ICAM-1 function means: (1) binding to the ICAM-1 protein to block its intrinsic function; (2) preventing interaction between the ICAM-1 protein and other molecules; (3) suppressing expression of ICAM-1, thereby eliminating its opportunity to function; or (4) directly or indirectly inhibiting ICAM-1 function.

In one embodiment, the ICAM-1 inhibitor may be an ICAM-1 neutralizing antibody. In another embodiment, the ICAM-1 inhibitor may be a nucleic acid molecule capable of silencing mRNA of the ICAM-1 gene. In particular, the nucleic acid molecule may be an antisense oligonucleotide, RNAi, siRNA, and/or shRNA. In another embodiment, the ICAM-1 inhibitor may be compounds which inhibit function of ICAM-1.

### Function of ICAM-1 inhibitor - Inhibition of ICAM-1-mediated signaling

The ICAM-1 inhibitor functions to suppress ICAM-1-mediated signaling. Although ICAM-1 is an adhesion molecule, and does not possess intrinsic enzymatic activity, it is known to activate intracellular signaling by binding to various ligands and signaling mediators. This is referred to as ICAM-1-mediated signaling (Lebedeva, Tatiana & Dustin, Michael & Sykulev, Yuri. (2005). ICAM-1 co-stimulates target cells to facilitate antigen presentation (Review). Current opinion in immunology. 17. 251-8). The ICAM-1-mediated signaling includes, for example, phosphorylation of multiple proteins including a transient phosphorylation of Src family kinases (e.g., p53/p56 lyn and pp60 src), an actin-binding protein p85 cortactin, a focal adhesion kinase (FAK), paxillin, and a 34 kDa cdc2, a cyclin-dependent kinase involved in cell cycle regulation (Greenwood et al., 2002; Hubbard and Rothlein, 2000). Signaling of pro-inflammatory cytokine such as NF-κB and Erk-1/Erk-2 MAPK is also included. The present inventors have revealed that ICAM-1 is involved in epithelial-to-mesenchymal transition (EMT) and accumulation of extracellular matrix (ECM), such as collagen, in fibrotic tissues. The present inventors have also revealed that inhibition of ICAM-1 can effectively suppress ICAM-1-mediated EMT and/or ICAM-1-mediated ECM aggregation.

### Therapeutic effect and mechanism of fibrosis treatment containing ICAM-1 inhibitor

The fibrosis treatment containing the ICAM-1 inhibitor according to the present disclosure, when administered to fibrotic tissues, exhibits the therapeutic effect of degrading accumulated extracellular matrix in the fibrotic tissues, and reverting the fibrotic tissues to normal tissues. The treatment containing the ICAM-1 inhibitor has a therapeutic mechanism that directly affects myofibroblasts to inactivate them. Specifically, the ICAM-1 inhibitor: (1) degrades extracellular matrix accumulated by myofibroblasts in fibrotic tissues; (2) inhibits secretion of extracellular matrix by myofibroblasts in fibrotic tissues; and (3) reduces myofibroblasts in fibrotic tissues, thereby treating fibrosis. The therapeutic effect and mechanisms described above are explained in more detail in the following sections.

### Advantage of present fibrosis treatment (1) - therapeutic effect on fibrosis

The fibrosis treatment containing the ICAM-1 inhibitor, when administered to fibrotic tissues, exhibits the following effects: (1) degradation of extracellular matrix accumulated by myofibroblasts, particularly collagen in fibrotic tissues; and (2) reduction of myofibroblasts in the fibrotic tissues, thereby reverting the fibrotic tissues to normal tissues. This indicates that the fibrosis treatment containing the ICAM-1 inhibitor provides a direct and complete therapeutic effect on fibrosis. The present inventors have confirmed through experiments that the accumulated extracellular matrix in fibrotic tissues is degraded, and then the fibrotic tissues revert to normal tissues. In contrast to conventional fibrosis treatments providing only an incomplete therapeutic effect on fibrosis, the fibrosis treatment containing the ICAM-1 inhibitor of the present disclosure demonstrates a complete therapeutic effect, and is therefore considered superior to conventional fibrosis treatments.

### Advantage of present fibrosis treatment (2) - therapeutic effect regardless of types of fibrotic tissues

The fibrosis treatment containing the ICAM-1 inhibitor has a therapeutic mechanism of directly acting on myofibroblasts which are main cause of fibrosis. Accordingly, the fibrosis treatment containing the ICAM-1 inhibitor can function as an effective treatment regardless of types of fibrotic tissues.

### Advantage of present fibrosis treatment (3) - low probability of adverse effects

The ICAM-1 is primarily expressed in a limited manner on vascular endothelial cells, and exhibits a low basal expression level in vivo. Moreover, principal function of the ICAM-1 as a cell adhesion molecule can be readily substituted or bypassed by other proteins of the same adhesion molecule family such as VCAM or ECAM. Accordingly, even when the fibrosis treatment containing the ICAM-1 inhibitor acts on ICAM-1 expressed in normal tissues or cells, (1) the low basal expression level of ICAM-1 minimizes likelihood of making problems because there are a few objects to be affected by the ICAM-1; (2) inhibition of ICAM-1 function can be readily substituted or bypassed by functions of other proteins; and (3) the fibrosis treatment containing the ICAM-1 inhibitor does not permanently suppress function and/or expression of the ICAM-1 in an individual's body, but rather transiently inhibits function and/or expression of the ICAM-1 in fibrotic tissues, so inhibitory effect of the ICAM-1 does not sustained beyond necessity. Therefore, the fibrosis treatment containing the ICAM-1 inhibitor is expected to exhibit a very low probability of adverse effects, even when administered to an individual's body.

### Therapeutic mechanism of fibrosis treatment containing ICAM-1 Inhibitor

### Therapeutic effect of fibrosis treatment

The fibrosis treatment containing the ICAM-1 inhibitor, as disclosed in the present disclosure, exhibits a direct therapeutic effect of reverting fibrotic tissues to normal tissues. Specifically, when administered to fibrotic tissues, the fibrosis treatment containing the ICAM-1 inhibitor: (1) removes abnormally accumulated extracellular matrix, particularly collagen, thereby reverting the extracellular matrix to a normal state; and (2) reduces aberrant myofibroblasts, resulting in reverting fibrotic tissues to normal tissues. The fibrosis treatment containing the ICAM-1 inhibitor exerts a therapeutic mechanism of inactivation of aberrantly activated myofibroblasts. The inactivation of the myofibroblasts includes (1) extracellular matrix remodeling mechanism; (2) an inhibitory mechanism of extracellular matrix production by myofibroblasts; and (3) reduction mechanism of myofibroblasts in fibrotic tissues. The fibrosis treatment containing the ICAM-1 inhibitor may inactivate myofibroblasts, and/or induce inactivation of the myofibroblasts.

### Therapeutic mechanism for fibrosis (1) - remodeling of extracellular matrix

The fibrosis treatment containing the ICAM-1 inhibitor removes extracellular matrix secreted and accumulated by myofibroblasts in fibrotic tissues, thereby reverting to extracellular matrix environment of normal tissues. In the present disclosure, this mechanism may be referred to as "extracellular matrix remodeling (ECM remodeling)". The extracellular matrix secreted and accumulated by myofibroblasts in fibrotic tissues may be collagen, fibronectin, hyaluronic acid, versican, and osteopontin (OPN), etc. The accumulation of the extracellular matrix interferes with normal function of tissues, and may be a cause of various pathological symptoms. In one embodiment, the extracellular matrix remodeling may be degradation of accumulated collagen in fibrotic tissues. A more detailed description of this therapeutic mechanism is provided in the section entitled "Therapeutic mechanism for fibrosis (1) - remodeling of extracellular matrix".

### Therapeutic mechanism for fibrosis (2) - Inhibition of extracellular matrix production by myofibroblasts

The fibrosis treatment containing the ICAM-1 inhibitor suppresses production and secretion of extracellular matrix by myofibroblasts in fibrotic tissues. As a result, extracellular matrix such as collagen secreted by myofibroblasts is no longer accumulated in the fibrotic tissues. This works in concert with the therapeutic mechanism (1) to eliminate abnormally accumulated extracellular matrix in fibrotic tissues, and revert the tissues to normal tissues. A more detailed explanation of this therapeutic mechanism is provided in the section entitled "Therapeutic mechanism for fibrosis (2) - Inhibition of extracellular matrix production by myofibroblasts".

### Therapeutic mechanism for fibrosis (3) - reduction of myofibroblasts in fibrotic tissues

The fibrosis treatment containing the ICAM-1 inhibitor reduces myofibroblasts present in fibrotic tissues. This reduction of myofibroblasts may result from one or more of the following mechanisms: (1) apoptosis of myofibroblasts in fibrotic tissues; (2) inhibition of differentiation of epithelial cells and/or residential fibroblasts into myofibroblasts in fibrotic tissues; and (3) reverting of myofibroblasts in fibrotic tissues to tissue epithelial cells, and/or residential fibroblasts. Through these mechanisms, the fibrotic tissues can be reversed to normal tissues. A more detailed explanation of this therapeutic mechanism is provided in the section entitled "Therapeutic mechanism for fibrosis (3) - reduction of myofibroblasts in fibrotic tissues".

### Combined action of therapeutic mechanisms

When the fibrosis treatment containing the ICAM-1 inhibitor is introduced into fibrotic tissues, the three aforementioned therapeutic mechanisms act in parallel, and/or in combination to treat fibrosis. These mechanisms may act sequentially, and/or simultaneously. The three therapeutic mechanisms have been categorized separately for the sake of explanatory convenience, and the actual pathways through which the ICAM-1 inhibitor exerts its effects may partially overlap.

### Therapeutic mechanism for fibrosis (1) - extracellular matrix remodeling

### Overview of extracellular matrix remodeling

The fibrosis treatment containing the ICAM-1 inhibitor, as provided in the present disclosure, is capable of reverting fibrotic tissues to normal tissues. Specifically, the fibrosis treatment containing the ICAM-1 inhibitor may be delivered to fibrotic tissues, and degrade extracellular matrix secreted and accumulated by myofibroblasts. This therapeutic mechanism is referred to as "extracellular matrix remodeling (ECM remodeling)." The extracellular matrix accumulated in fibrotic tissues may include, for example, collagen, fibronectin, hyaluronic acid, versican, and osteopontin (OPN), etc. When the fibrosis treatment containing the ICAM-1 inhibitor is administrated to fibrotic tissues, the fibrosis treatment removes extracellular matrix secreted and accumulated by myofibroblasts, through various pathways. In one embodiment, the fibrosis treatment containing the ICAM-1 inhibitor may promote secretion and/or activation of matrix metalloproteinase (MMP) in tissues. In another embodiment, the fibrosis treatment containing the ICAM-1 inhibitor may suppress secretion and/or activation of tissue inhibitor of metalloproteinase (TIMP) within tissues. In yet another embodiment, the fibrosis treatment containing the ICAM-1 inhibitor may inhibit secretion and/or activation of Hyaluronan Synthase 3 (HAS3) in tissues.

### Exemplary pathway of extracellular matrix remodeling 1 - promotion of MMP secretion

Matrix metalloproteinase (MMP), also referred to as matrixin, are a class of proteinase involved in degradation of extracellular matrix. Accordingly, when MMP is secreted and activated in tissues, it degrades accumulated extracellular matrix, particularly collagen. In one embodiment, the fibrosis treatment containing the ICAM-1 inhibitor may promote secretion and/or activation of MMP in fibrotic tissues. Specifically, the MMP may be one or more selected from a group consisting of MMP2, MMP9, and MMP13.

### Exemplary pathway of extracellular matrix remodeling 2 - inhibition of TIMP secretion

As a name suggests, tissue inhibitor of matrix metalloproteinase (TIMP) is a protein that suppress activity of the matrix metalloproteinase (MMP). The TIMP regulates activity of MMP described above, and specifically, functions to inhibit enzymatic activity of MMP. Accordingly, when secretion and/or activation of the TIMP are/is suppressed in tissues, the activity of MMP increases, thereby degrading accumulated extracellular matrix.

### Therapeutic mechanism for fibrosis (2) - inhibition of extracellular matrix production by myofibroblasts

### Overview of extracellular matrix secretion

The fibrosis treatment containing the ICAM-1 inhibitor, as provided in the present disclosure, is capable of suppressing production and secretion of extracellular matrix by myofibroblasts in fibrotic tissues. The extracellular matrix may be one or more selected from a group consisting of collagen, fibronectin, hyaluronic acid, versican, and osteopontin (OPN). In order to prevent further extracellular matrix accumulation in fibrotic tissues, it is required to inhibit production and secretion of extracellular matrix by myofibroblasts. When the fibrosis treatment containing the ICAM-1 inhibitor is administered to fibrotic tissues, extracellular matrix production and secretion by myofibroblasts through various pathways are inhibited. In one embodiment, the fibrosis treatment containing the ICAM-1 inhibitor may suppress expression and secretion of collagen and/or osteopontin (OPN) by myofibroblasts in fibrotic tissues. In another embodiment, the fibrosis treatment containing the ICAM-1 inhibitor may inhibit production of hyaluronan synthase (HAS) by myofibroblasts in fibrotic tissues, thereby suppressing synthesis and secretion of hyaluronic acid.

### Exemplary pathway of inhibition of extracellular matrix secretion (1) - suppression of collagen expression

One of the most actively secreted extracellular matrix by myofibroblasts in fibrotic tissues is collagen. Therefore, in addition to removing already accumulated collagen, it is also important to block expression and secretion of the collagen by the myofibroblasts. The fibrosis treatment containing the ICAM-1 inhibitor according to the present disclosure is capable of suppressing expression and secretion of collagen by myofibroblasts in fibrotic tissues. As demonstrated in Experimental Examples 3 to 7, when the fibrosis treatment containing the ICAM-1 inhibitor of the present disclosure, is administered to fibrotic tissues and/or cells, it can be found that reduction in collagen expression in myofibroblasts is accomplished.

### Exemplary pathway of inhibition of extracellular matrix secretion (2) - suppression of HAS expression

Hyaluronan synthase (HAS) is an enzyme responsible for synthesis of hyaluronic acid, and is involved in hyaluronic acid synthesis at plasma membranes. Accordingly, when expression of HAS is suppressed in cells, synthesis and secretion of hyaluronic acid are inhibited, thereby preventing myofibroblasts from producing hyaluronic acid. The HAS may be one or more selected from a group consisting of HAS1, HAS2, and HAS3.

### Exemplary pathway of inhibition of extracellular matrix secretion (3) - suppression of OPN expression

Osteopontin (OPN) is a major phosphorylated protein composing skeletons, and is responsible for skeletal remodeling. In addition, it is known that OPN is associated with onset and progression of tissue fibrosis. The fibrosis treatment containing the ICAM-1 inhibitor according to the present disclosure, is capable of suppressing expression and secretion of OPN by myofibroblasts in fibrotic tissues. As demonstrated in Experimental Example 3.6 and FIG. (), when the fibrosis treatment containing the ICAM-1 inhibitor of the present disclosure is administered to fibrotic tissues and/or cells, it is confirmed that reduction in OPN expression in myofibroblasts is accomplished.

### Therapeutic mechanism for fibrosis (3) - reduction of myofibroblasts in fibrotic tissues

### Overview of reduction of myofibroblasts in fibrotic tissues

Fibrosis treatment containing the ICAM-1 inhibitor, as provided in the present disclosure, is capable of reducing myofibroblasts in fibrotic tissues. This reduction of myofibroblasts may result from one or more of the following mechanisms: (1) apoptosis of myofibroblasts in fibrotic tissues; (2) inhibition of differentiation of epithelial cells and/or residential fibroblasts into myofibroblasts in fibrotic tissues; and (3) reversion of myofibroblasts into epithelial cells and/or residential fibroblasts in fibrotic tissues. As a result, the fibrotic tissues can be reversed to normal tissues. This myofibroblast-reducing effect in fibrotic tissues is well demonstrated in Experimental Examples 3.3 and 6.2.

### Reduction of myofibroblasts through apoptosis

As described above, when the fibrosis treatment containing the ICAM-1 inhibitor is administered to fibrotic tissues, myofibroblasts in fibrotic tissues will be inactivated. As a result, the myofibroblasts undergo apoptosis, leading to reduction of myofibroblasts in tissues.

### Inhibition of epithelial-to-mesenchymal transition of epithelial cells in tissues

The fibrosis treatment containing the ICAM-1 inhibitor may also suppress epithelial-to-mesenchymal transition (EMT) which transdifferentiates normal epithelial cells into mesenchymal cells in fibrotic tissues. As described above, when the epithelial-to-mesenchymal transition is inhibited, myofibroblasts will not increase any more, since normal epithelial cells undergo transdifferentiation into mesenchymal cells through the epithelial-to-mesenchymal transition.

### Inhibition of differentiation of residential fibroblasts into myofibroblasts in tissues

The fibrosis treatment containing the ICAM-1 inhibitor may also suppress differentiation of residential fibroblasts into myofibroblasts in fibrotic tissues. As described above, when this differentiation of residential fibroblasts into myofibroblasts is inhibited, myofibroblasts will not increase any more, since the resident myofibroblasts undergo transdifferentiation into myofibroblasts during progression of fibrosis.

### Reversion of myofibroblasts into tissue-resident epithelial cells

The fibrosis treatment containing the ICAM-1 inhibitor may induce and promote reversion of the myofibroblasts to epithelial cells in fibrotic tissues. As described above, epithelial cells can undergo epithelial-to-mesenchymal transition (EMT) and differentiate into myofibroblasts. Conversely, reverse process of mesenchymal-to-epithelial transition (MET) may occur, allowing myofibroblasts to revert to epithelial cells of normal tissues. This mechanism may act in concert with reduction of myofibroblasts via apoptosis, and inhibition of transdifferentiation of epithelial cell into myofibroblasts. As shown in Experimental Example 4 and related FIGS, there is indirect evidence supporting reversion of the myofibroblasts to epithelial cells.

### Reversion of myofibroblasts into residential fibroblasts

The fibrosis treatment containing the ICAM-1 inhibitor may induce and promote reversion of the myofibroblasts to residential fibroblasts. As described above, the residential fibroblasts can differentiate into myofibroblasts during the progression of fibrosis. Conversely, the reverse process may occur, whereby myofibroblasts revert to residential fibroblasts. This reversion may act in concert with myofibroblast reduction via apoptosis, and inhibition of differentiation of residential fibroblast into myofibroblasts.

### Biomarkers associated with inhibition of epithelial-to-mesenchymal transition, or reversion of myofibroblasts to tissue epithelial cells, and/or residential fibroblasts

Biomarkers associated with epithelial-to-mesenchymal transition (EMT), or reversion of myofibroblasts to epithelial cells include E-cadherin, Snaill, Snail2, Zeb1, Zeb2, vimentin, N-cadherin, and fibronectin. Specifically, when epithelial-to-mesenchymal transition occurs, expression level of E-cadherin may decrease, and/or expression levels of one or more markers selected from Snaill, Snail2, Zeb1, Zeb2, vimentin, N-cadherin, and fibronectin may increase. Conversely, when expression level of E-cadherin increases, and/or expression levels of one or more markers selected from Snaill, Snail2, Zeb1, Zeb2, vimentin, N-cadherin, and fibronectin decrease, it may be interpreted as: 1) epithelial-to-mesenchymal transition is suppressed, thereby preventing further differentiation into myofibroblasts; or 2) myofibroblasts revert to tissue epithelial cells.

### Active ingredient 1 of fibrosis treatment - ICAM-1 neutralizing antibody or fragments thereof

### Overview of ICAM-1 neutralizing antibody or fragments thereof

The fibrosis treatment disclosed in the present disclosure contains the ICAM-1 inhibitor, wherein the ICAM-1 inhibitor may be an ICAM-1 neutralizing antibody or fragments thereof. The ICAM-1 neutralizing antibody or fragments thereof binds to the ICAM-1 protein expressed in fibrotic tissues, and interferes with interaction between the ICAM-1 protein and other molecules, thereby inhibiting function of the ICAM-1 protein. The ICAM-1 neutralizing antibody or fragments thereof is not particularly limited, as long as it is capable of inhibiting ICAM-1. The term "fragments of the ICAM-1 neutralizing antibody" refers to fragments exhibiting antigen-binding function, and include: (1) a portion of an amino acid sequence of the ICAM-1 neutralizing antibody; (2) various combinations of one or more fragments of the ICAM-1 neutralizing antibody; and (3) combinations of one or more fragments of the ICAM-1 neutralizing antibody, and additional necessary molecules.

### Binding target 1 of ICAM-1 neutralizing antibody or fragments thereof - ICAM-1

The ICAM-1 neutralizing antibody or fragments thereof may bind to ICAM-1 present in a human body. The ICAM-1 includes not only transmembrane proteins expressed on vascular endothelial cells or leukocytes, but also a portion of ICAM-1 that circulates freely in bloodstream due to cleavage of certain region of the ICAM-1 protein. In one embodiment, the ICAM-1 neutralizing antibody or fragments of the ICAM -1 neutralizing antibody may bind to all or part of a protein represented by an amino acid sequence as below:

MAPSSPRPALPALLVLLGALFPGPGNAQTSVSPSKV
ILPRGGSVLVTCSTSCDQPKLLGIETPLPKKELLLPGNNR
KVYELSNVQEDSQPMCYSNCPDGQSTAKTFLTVYWTPERV
ELAPLPSWQPVGKNLTLRCQVEGGAPRANLTVVLLRGEKE
LKREPAVGEPAEVTTTVLVRRDHHGANFSCRTELDLRPQG
LELFENTSAPYQLQTFVLPATPPQLVSPRVLEVDTQGTVV
CSLDGLFPVSEAQVHLALGDQRLNPTVTYGNDSFSAKASV
SVTAEDEGTQRLTCAVILGNQSQETLQTVTIYSFPAPNVI
LTKPEVSEGTEVTVKCEAHPRAKVTLNGVPAQPLGPRAQL
LLKATPEDNGRSFSCSATLEVAGQLIHKNQTRELRVLYGP
RLDERDCPGNWTWPENSQQTPMCQAWGNPLPELKCLKDGT
FPLPIGESVTVTRDLEGTYLCRARSTQGEVTRKVTVNVLS
PRYEIVIITVVAAAVIMGTAGLSTYLYNRQRKIKKYRLQQ
AQKGTPMKPNTQATPP (SEQ ID NO:1)

In another embodiment, the ICAM-1 neutralizing antibody or fragments of the ICAM-1 neutralizing antibody may bind to all or part of a protein represented by an amino acid sequence as below:

QTSVSPSKVILPRGGSVLVTCSTSCDQPKLLGIETPLPKKELLLPGNNRK
VYELSNVQEDSQPMCYSNCPDGQSTAKTFLTVYWTPERVE
LAPLPSWQPVGKNLTLRCQVEGGAPRANLTVVLLRGEKEL
KREPAVGEPAEVTTTVLVRRDHHGANFSCRTELDLRPQGL
ELFENTSAPYQLQTFVLPATPPQLVSPRVLEVDTQGTVVC
SLDGLFPVSEAQVHLALGDQRLNPTVTYGNDSFSAKASVS
VTAEDEGTQRLTCAVILGNQSQETLQTVTIYSFPAPNVIL
TKPEVSEGTEVTVKCEAHPRAKVTLNGVPAQPLGPRAQLL
LKATPEDNGRSFSCSATLEVAGQLIHKNQTRELRVLYGPR
LDERDCPGNWTWPENSQQTPMCQAWGNPLPELKCLKDGTF
PLPIGESVTVTRDLEGTYLCRARSTQGEVTRKVTVNVLSPRYE (SEQ ID NO:2)

### Target for binding to ICAM-1 neutralizing antibody or fragments thereof (2) - epitope of ICAM-1

The ICAM-1 neutralizing antibody or fragments of the ICAM-1 neutralizing antibody must have function of inhibiting the ICAM-1, and therefore must be able to bind to portions of ICAM-1 that can interact with other molecules. In other words, the epitope of ICAM-1 to which the ICAM-1 neutralizing antibody or fragments of the ICAM-1 neutralizing antibody can bind is a portion directly or indirectly related to function of ICAM-1. In one embodiment, the ICAM-1 neutralizing antibody or fragments of the ICAM-1 neutralizing antibody may bind to an extracellular domain of the ICAM-1 protein. Specifically, the extracellular domain may be one or more domain selected from D1, D2, D3, D4, and D5 domains.

### ICAM-1 neutralizing antibody 1 - Classification by class

The ICAM-1 neutralizing antibody includes all isotypes or classes of antibodies found in humans or mammalian animals. In one embodiment, the ICAM-1 neutralizing antibody may belong to a class selected from immunoglobulin A (IgA), immunoglobulin D (IgD), immunoglobulin E (IgE), immunoglobulin G (IgG), and immunoglobulin M (IgM). As an example, when the ICAM-1 neutralizing antibody is IgA, it may be a subclass selected from IgA1 and IgA2. As another example, when the ICAM-1 neutralizing antibody is IgG, it may be a subclass selected from IgG1, IgG2, IgG3, and IgG4.

### ICAM-1 neutralizing antibody 3 - Classification by form

In one embodiment, the ICAM-1 neutralizing antibody may be a polyclonal antibody. In one embodiment, the ICAM-1 neutralizing antibody may be a monoclonal antibody. As an example, the ICAM-1 neutralizing antibody may be a hybridoma monoclonal antibody. As another example, the ICAM-1 neutralizing antibody may be a recombinant monoclonal antibody.

### ICAM-1 neutralizing antibody 2 - Classification by origin

The ICAM-1 neutralizing antibody is preferably derived from humans, but it is also acceptable when all or parts of the ICAM-1 neutralizing antibody is derived from non-human animals, as long as it exhibits therapeutic efficacy in humans and does not cause fatal side effects. In one embodiment, the ICAM-1 neutralizing antibody may be a human-derived antibody. In another embodiment, the ICAM-1 neutralizing antibody may be a humanized antibody. Here, the humanized antibody may be an antibody in which a hypervariable region is derived from a non-human animal, and remaining parts are derived from a human. In another embodiment, the ICAM-1 neutralizing antibody may be a chimeric antibody. Here, the chimeric antibody may be an antibody in which a variable region is derived from a non-human animal, and remaining parts are derived from a human. In another embodiment, the ICAM-1 neutralizing antibody may be an antibody derived from a non-human animal. In one embodiment, the non-human animal may be a mammal. Specifically, it may be a mouse, but is not limited thereto.

### Fragments of ICAM-1 neutralizing antibody

Fragments of the ICAM-1 neutralizing antibody is not particularly limited as long as it can inhibit function of the ICAM-1. Fragments of the ICAM-1 neutralizing antibody includes: 1) a portion of an amino acid sequence of the ICAM-1 neutralizing antibody, 2) various combinations of one or more fragments of the ICAM-1 neutralizing antibody, and 3) combinations of one or more fragments of the ICAM-1 neutralizing antibody, and additional necessary molecules. In one embodiment, fragments of the ICAM-1 neutralizing antibody may be selected from Fab, monospecific F(ab)₂, bispecific F(ab)₂, F(ab')₂, monovalent antibody, ScFv (single-chain variable fragment), dAb, monospecific diabody, bispecific diabody, minibody, and ScFv-Fc.

### Example 1 of ICAM-1 neutralizing antibody or fragments thereof - known antibody or fragments thereof

The ICAM-1 neutralizing antibody may be known neutralizing antibody or fragments thereof that are readily available to those skilled in the art.

### Example 2 of ICAM-1 neutralizing antibody or fragments thereof - selected ICAM-1 antibody or fragments thereof

In one embodiment, the ICAM-1 antibody or fragments thereof may be an ICAM-1 neutralizing antibody and fragments thereof selected by known methods. As an example of the known methods, a phage display formation method is described in section as below, titled "Method of selecting ICAM-1 neutralizing antibody".

### Method of selecting ICAM-1 neutralizing antibody

### Overview of method of selecting ICAM-1 neutralizing antibody

A method of selecting ICAM-1 neutralizing antibody is not limited to any particular method, as long as it can select antibodies capable of inhibiting ICAM-1. Therefore, the ICAM-1 neutralizing antibody can be selected using any appropriate known method, or by commissioning manufacturers specializing in selection of neutralizing antibodies. The following description is only an example of a method of selecting neutralizing antibodies using a phage display method, and a bio-panning method, which are widely used methods of selecting neutralizing antibodies, and a method of selecting the ICAM-1 neutralizing antibody disclosed herein is not limited to the following examples.

### Selection of ICAM-1 neutralizing antibody candidates through phage display method

Antibodies that can bind to the ICAM-1 protein can be selected using the phage display method. The specific process includes the following steps:
(1) Using all antibody genes in a human body, obtain phage display libraries that express antibody fragments (ScFvs) containing variable regions of the antibody genes, on a surface.
(2) Expose the ICAM-1 protein to the phage display libraries, and use a bio-panning method to identify only ScFvs that bind to ICAM-1. Here, the bio-panning method is not particularly limited as long as it can identify only ScFvs that bind to the ICAM-1 protein, and known bio-panning methods can be used.
(3) Repeat (2) 3-4 times to increase accuracy.
(4) Analyze sequences of ScFvs selected in (3) to select candidate sequences of the ICAM-1 neutralizing antibodies.

### Optimization of CDR sequences of selected ICAM-1 neutralizing antibodies

For each of the selected ICAM-1 neutralizing antibody sequences, libraries are created by randomly changing one or more complementarity determining region (CDR) sequences. Specifically, the libraries may include: 1) parent ICAM-1 neutralizing antibody (hereinafter referred to as parent antibody) sequences, 2) sequences in which one or more amino acids in CDR sequences of the parent antibody are replaced/modified with any amino acids, 3) sequences newly constructed by randomly rearranging positions of CDR sequences of heavy chain and light chain of the parent antibody, and/or 4) appropriately combined sequences of 2) and 3). Subsequently, the libraries is appropriately subjected to ELISA testing and biopanning method to select sequences with a highest binding affinity to ICAM-1, followed by optimizing the CDR sequences of the ICAM-1 neutralizing antibody candidate sequences based on this result.

### Verification of the ICAM-1-mediated signaling inhibitory ability of selected ICAM-1 antibody

The ICAM-1-mediated signaling inhibitory ability of the selected ICAM-1 antibody is verified by the following methods:
(1) In vitro, epithelial cell lines of various tissues are stimulated with TGF-β to confirm development of myofibroblasts, secretion of extracellular matrix, and so on.
(2) For each of the epithelial cell lines, cells (a control group) to which only TGF-β has been added, are compared with cells (an experimental group) to which TGF-β and the selected ICAM-1 neutralizing antibody candidates have been administered.
(3) As disclosed by the present inventors, when the ICAM-1 neutralizing antibody is added, fibrosis of the epithelial cells is inhibited, and the therapeutic effect is observed. Therefore, the experimental group is compared with the control group in order to confirm whether fibrosis of the cell lines progresses or not.
(4) The ICAM-1 neutralizing antibody candidates that show effective fibrosis inhibition, and the therapeutic effect, were selected based on results of the confirmation.

### Effective ingredient of fibrosis treatment 2 - other ICAM-1 inhibitors

### Overview of other ICAM-1 inhibitors

The ICAM-1 inhibitors disclosed herein are not limited as long as it can inhibit functions of ICAM-1. As mentioned above, the ICAM-1 inhibitors may 1) interfere with interaction between the ICAM-1 protein and other molecules, or 2) inhibit expression of the ICAM-1 protein, thereby reducing absolute amount of the ICAM-1 protein that interacts with other molecules, and ultimately interfering with functions of the ICAM-1 protein. Examples of ICAM-1 inhibitor capable of the above actions include nucleic acids that silence mRNA of the ICAM-1 gene, CRISPR/Cas systems that knockout or knockdown the ICAM-1 gene, compounds that inhibit the ICAM-1 protein, or aptamers that inhibit the ICAM-1 protein, but are not limited to these.

### Nucleic acids that silence mRNA of ICAM-1 gene

The ICAM-1 inhibitor may silence mRNA of the ICAM-1 gene to interfere with translation of the ICAM-1 protein. In one embodiment, the ICAM-1 inhibitor is a nucleic acid that specifically binds to mRNA transcribed from the ICAM-1 gene, and may be selected from small interfering RNA (siRNA), small hairpin RNA (shRNA), and antisense nucleic acids.

### CRISPR/Cas system 1 targeting ICAM-1 gene - nuclease

The ICAM-1 inhibitor may knock out the ICAM-1 gene in cellular genome to prevent production of the ICAM-1 protein. For example, a CRISPR/Cas system that acts as a target-specific nuclease can cleave specific genes in cellular genome, induce errors in the cellular gene repair mechanisms (e.g., non-homologous end joining (NHEJ), and/or homologous recombination repair (HDR)) to generate an indel in the specific gene, thereby knocking out the specific gene. In one embodiment, the ICAM-1 inhibitor may be a CRISPR/Cas system (composition) including: a Cas protein or nucleic acid encoding the same; and a guide RNA or nucleic acid encoding the same, wherein the guide RNA is capable of targeting the ICAM-1 gene in cellular genome, and interacting with the Cas protein to form a complex.

### CRISPR/Cas system 2 targeting ICAM-1 gene - regulator

The ICAM-1 inhibitor may induce a knockdown effect of suppressing expression of the ICAM-1 gene in cellular genome, thereby inhibiting production of the ICAM-1 protein. For example, as a target-specific embodiment, the ICAM-1 inhibitor may be a CRISPRi system (composition) including: a fusion protein including a Cas protein, and at least one gene expression regulatory domains, or a nucleic acid encoding the fusion protein; and guide RNA, and a nucleic acid encoding the guide RNA, wherein the guide RNA is capable of targeting the ICAM-1 gene in cellular genome, and interacting with the fusion protein to form a complex.

### Substance 1 that inhibits ICAM-1 protein - compounds

In one embodiment, the ICAM-1 inhibitor may be compounds, and/or small molecules that function to prevent the ICAM-1 protein from interacting with other molecules.

### Substance 2 that inhibits ICAM-1 protein - aptamers

In one embodiment, the ICAM-1 inhibitor may be DNA and/or RNA aptamer that function(s) to prevent the ICAM-1 protein from interacting with other molecules.

### Pharmaceutical composition for treating fibrosis

### Overview of pharmaceutical composition for treating fibrosis

The present disclosure provides a pharmaceutical composition for treating fibrosis, the pharmaceutical composition including the ICAM-1 inhibitor. The pharmaceutical composition for treating fibrosis may include a therapeutically effective amount of the ICAM-1 inhibitor; pharmaceutically acceptable carriers; and/or adjuvants. Here, the pharmaceutically acceptable carriers may be intended for appropriate formulation of the pharmaceutical composition for treating fibrosis.

### Target disease - fibrosis

The pharmaceutical composition for treating fibrosis is intended for treating fibrosis, or fibrosis-associated diseases, disorders, and/or symptoms. The fibrosis or fibrosis-associated diseases, disorders, and/or symptoms include all those recognizable by those skilled in the art. In one embodiment, the fibrosis may be as described in the section titled "Pathogenesis of fibrosis." In one embodiment, the fibrosis or fibrosis-associated diseases, disorders, and/or symptoms may be selected from a group consisting of hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; chronic pancreatitis; duct obstruction; autoimmune-interstitial pulmonary disease such as ankylosing spondylitis, and rheumatoid arthritis; connective tissue disease-interstitial pulmonary disease such as rheumatoid arthritis-interstitial pulmonary disease; and non-idiopathic pulmonary fibrosis-interstitial pulmonary disease.

### Exemplary formulation of pharmaceutical composition for treating fibrosis

In one embodiment, the pharmaceutical composition for treating fibrosis, which includes the ICAM-1 inhibitor may be formulated as troches, lozenges, tablets, water-soluble suspensions, oil-soluble suspensions, powder formulations, granules, emulsions, hard capsules, soft capsules, syrups, or elixirs. In another embodiment, the pharmaceutical composition for treating fibrosis, which includes the ICAM-1 inhibitor may be formulated as an injectable solution, suppository, respiratory inhalation powder, spray aerosol, ointment, topical powder, oil, or cream. In another embodiment, the pharmaceutical composition for treating fibrosis, including the ICAM-1 inhibitor may be formulated as an injectable solution. Specifically, a therapeutically effective amount of the ICAM-1 inhibitor may be mixed with stabilizers or buffers in water to prepare a solution or suspension, and the solution or suspension may be formulated into unit doses in ampoules or vials. In another embodiment, the pharmaceutical composition for treating fibrosis, which includes the ICAM-1 inhibitor may be formulated as an aerosol by mixing the ICAM-1 inhibitor with a propellant, and other additives to produce water-dispersible concentrate, or wettable powder. In another embodiment, when the pharmaceutical composition for treating fibrosis, which includes the ICAM-1 inhibitor is formulated for transdermal use, therapeutically effective amount of the ICAM-1 inhibitor can be combined with animal oil, vegetable oil, wax, paraffin, starch, tragacanth, cellulose derivatives, polyethylene glycol, silicone, bentonite, silica, talc, zinc oxide, etc. as carriers, to produce ointments, creams, topical powders, oils, and topical preparations.

### Examples of pharmaceutically acceptable carriers

Pharmaceutically acceptable carriers are those commonly used in formulations, such as saline solution, sterile water, Ringer's solution, buffered saline solution, cyclodextrin, dextrose solution, maltodextrin solution, glycerol, ethanol, liposomes, etc., but are not limited thereto, and may additionally include other conventional additives such as antioxidants and buffers as needed. Moreover, diluents, dispersants, surfactants, binders, lubricants, etc., may be added additionally to formulate the product into injectable formulations such as aqueous solutions, suspensions, emulsions, pills, capsules, granules, or tablets. Regarding suitable pharmaceutically acceptable carriers and formulations, each component can be formulated appropriately using methods described in Remington's Pharmaceutical Sciences (19th edition, 1995). In one embodiment, the pharmaceutical composition for treating fibrosis, which includes the ICAM-1 inhibitor may be formulated using pharmaceutically acceptable carriers such as binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol (PEG) wax; sweeteners; flavoring agents; syrups; liquid carriers such as fatty oils; sterile aqueous solutions; propylene glycol; polyethylene glycol; injectable esters such as ethyl oleate; suspending agents; emulsifiers; lyophilized preparations; topical formulations; stabilizers; buffers; animal oils; vegetable oils; waxes; paraffin; starches; tragacanth; cellulose derivatives; polyethylene glycol; silicone; bentonite; silica; talc; zinc oxide; and appropriate combinations thereof.

### Method of treating fibrosis

### Overview of method of treating fibrosis

The present disclosure provides a method of treating fibrosis using a fibrosis treatment containing the ICAM-1 inhibitor. The method of treating fibrosis includes administering the pharmaceutical composition for treating fibrosis, the pharmaceutical composition appropriately being formulated with a therapeutically effective ICAM-1 inhibitor, to a subject using an appropriate administration method, appropriate administration regime, and appropriate administration dosage.

### Target disease - fibrosis

The fibrosis is the same as that described in the subsection "Target disease - fibrosis" of paragraph "Pharmaceutical composition for treating fibrosis."

### Examples of administration method

The method of treating fibrosis may involve administering the pharmaceutical composition appropriately being formulated with a therapeutically effective ICAM-1 inhibitor to a subject using an appropriate administration method. In one embodiment, the method of treating fibrosis may involve administering the pharmaceutical composition appropriately being formulated with a therapeutically effective ICAM-1 inhibitor to a subject using one of the following administration methods: oral administration, non-oral administration, intravenous administration, intraperitoneal administration, intramuscular administration, transdermal administration, or subcutaneous administration.

### Examples of administration dosage

The method of treating fibrosis may involve administering the pharmaceutical composition appropriately being formulated with a therapeutically effective ICAM-1 inhibitor to s subject at an appropriate dosage. In one embodiment, the dosage may be approximately 0.01 mg to 1000 mg per 1 kg of body weight of a subject, with respect to the ICAM-1 inhibitor.

### Examples of administration schedule

The method of treating fibrosis may involve administering the pharmaceutical composition appropriately being formulated with a therapeutically effective ICAM-1 inhibitor to a subject at an appropriate administration schedule. In one embodiment, the pharmaceutical composition appropriately being formulated with a therapeutically effective ICAM-1 inhibitor may be administered once daily. In another embodiment, the pharmaceutical composition appropriately being formulated with a therapeutically effective ICAM-1 inhibitor may be administered two or more times per day. In yet another embodiment, the pharmaceutical composition appropriately being formulated with a therapeutically effective ICAM-1 inhibitor may be administered at intervals of 1 hour, 2 hours, 6 hours, 12 hours, 24 hours, 2 days, 3 days, one week, two weeks, one month, and/or three months.

### Examples of the present disclosure

The following are examples of possible embodiments of the invention provided herein. The embodiments provided in this paragraph are merely examples of the disclosure. Therefore, the disclosure provided herein cannot be interpreted as being limited to the following examples. The brief descriptions provided with example numbers are also for the convenience of distinguishing between each example, and cannot be interpreted as limiting the disclosure disclosed herein.

### Fibrosis

Example 1. Fibrosis
   one or more of the following diseases, disorders, and/or symptoms:
   fibrosis; and fibrosis-associated diseases.
Example 2. Myofibroblast-related fibrosis
   In Example 1, the fibrosis is induced by myofibroblasts.
Example 3. Fibrotic tissues
   In Example 1 or 2, the fibrosis develops in one or more of the following tissues:
   skin; brain; nervous system; heart; bone marrow; liver; gut; joint; eye; lung; kidney; retroperitoneum; mediastinum; and pancreas.
Example 4. Specification of fibrosis-associated diseases
   In any one of Examples 1 to 3, the disease, disorder, and/or symptom is one or more selected from the following:
   hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; chronic pancreatitis; duct obstruction; autoimmune-interstitial pulmonary disease such as ankylosing spondylitis, and rheumatoid arthritis; connective tissue disease-interstitial pulmonary disease such as rheumatoid arthritis-interstitial pulmonary disease; and non-idiopathic pulmonary fibrosis-interstitial pulmonary disease.

### ICAM-1 inhibitor - ICAM-1 neutralizing antibody

### Example 5. ICAM-1 neutralizing antibody

### ICAM-1 neutralizing antibody

### Example 6. Subject neutralizing antibody

In Example 5, the ICAM-1 neutralizing antibody is an antibody capable of neutralizing the ICAM-1 protein represented by at least one amino acid sequence selected from:

MAPSSPRPALPALLVLLGALFPGPGNAQTSVSPSKV
ILPRGGSVLVTCSTSCDQPKLLGIETPLPKKELLLPGNNR
KVYELSNVQEDSQPMCYSNCPDGQSTAKTFLTVYWTPERV
ELAPLPSWQPVGKNLTLRCQVEGGAPRANLTVVLLRGEKE
LKREPAVGEPAEVTTTVLVRRDHHGANFSCRTELDLRPQG
LELFENTSAPYQLQTFVLPATPPQLVSPRVLEVDTQGTVV
CSLDGLFPVSEAQVHLALGDQRLNPTVTYGNDSFSAKASV
SVTAEDEGTQRLTCAVILGNQSQETLQTVTIYSFPAPNVI
LTKPEVSEGTEVTVKCEAHPRAKVTLNGVPAQPLGPRAQL
LLKATPEDNGRSFSCSATLEVAGQLIHKNQTRELRVLYGP
RLDERDCPGNWTWPENSQQTPMCQAWGNPLPELKCLKDGT
FPLPIGESVTVTRDLEGTYLCRARSTQGEVTRKVTVNVLS
PRYEIVIITVVAAAVIMGTAGLSTYLYNRQRKIKKYRLQQ
AQKGTPMKPNTQATPP (SEQ ID NO:1)
and
QTSVSPSKVILPRGGSVLVTCSTSCDQPKLLGIETPLPKKELLLPGNNRK
VYELSNVQEDSQPMCYSNCPDGQSTAKTFLTVYWTPERVE
LAPLPSWQPVGKNLTLRCQVEGGAPRANLTVVLLRGEKEL
KREPAVGEPAEVTTTVLVRRDHHGANFSCRTELDLRPQGL
ELFENTSAPYQLQTFVLPATPPQLVSPRVLEVDTQGTVVC
SLDGLFPVSEAQVHLALGDQRLNPTVTYGNDSFSAKASVS
VTAEDEGTQRLTCAVILGNQSQETLQTVTIYSFPAPNVIL
TKPEVSEGTEVTVKCEAHPRAKVTLNGVPAQPLGPRAQLL
LKATPEDNGRSFSCSATLEVAGQLIHKNQTRELRVLYGPR
LDERDCPGNWTWPENSQQTPMCQAWGNPLPELKCLKDGTF
PLPIGESVTVTRDLEGTYLCRARSTQGEVTRKVTVNVLSPRYE (SEQ ID NO:2)

### Example 7. Specification of epitopes

In Example 6, the ICAM-1 neutralizing antibody is an antibody capable of binding to an extracellular domain.

### Example 8. Additional specification of domains

In Example 7, the ICAM-1 neutralizing antibody is an antibody capable of binding to at least one domain selected from D1, D2, D3, D4, and D5 domains.

### ICAM-1 inhibitor 2 - fragments of ICAM-1 neutralizing antibody

### Example 9. Fragments of ICAM-1 neutralizing antibody

Fragments of the ICAM-1 neutralizing antibody includes at least one components selected from the following:
a part of any one of the ICAM-1 neutralizing antibodies of Examples 5 to 8;
a combination of one or more parts of the ICAM-1 neutralizing antibodies; and
a combination of one or more parts of the ICAM-1 neutralizing antibodies, and additional molecules,
wherein the fragments of the ICAM-1 neutralizing antibody is capable of neutralizing ICAM-1.

### Example 3. Examples of fragments of ICAM-1 neutralizing antibody

In Example 9, the fragments of the ICAM-1 neutralizing antibody is selected from the following:
Fab; monospecific F(ab)₂; bispecific F(ab)₂; F(ab')₂; monovalent antibody; ScFv; dAb; monospecific diabody; bispecific diabody; minibody; and ScFv-Fc.

### ICAM-1 Inhibitor 3 - nucleic acid that silences mRNA of ICAM-1 gene

### Example 11. Nucleic acid that silences mRNA of ICAM-1

A nucleic acid capable of silencing the mRNA of the ICAM-1 gene.

### Example 12. Examples of nucleic acid

In Example 11, the nucleic acid is selected from the following:
small interfering RNA (siRNA); small hairpin RNA (shRNA); and antisense nucleic acid.

### Example 13. Examples of nucleic acid target

In Example 12, the nucleic acid has a sequence complementary to all or part of the mRNA of the ICAM-1 gene.

### Example 14. Example of mRNA

In Example 13, the mRNA of the ICAM-1 gene is represented by the following nucleic acid sequence:

CGCTGAATGGGGTTCCAGCCCAGCCACTGGGCCCGAGGGC
CCAGCTCCTGCTGAAGGCCACCCCAGAGGACAACGGGCGC
AGCTTCTCCTGCTCTGCAACCCTGGAGGTGGCCGGCCAGC
TTATACACAAGAACCAGACCCGGGAGCTTCGTGTCCTGTA
TGGCCCCCGACTGGACGAGAGGGATTGTCCGGGAAACTGG
ACGTGGCCAGAAAATTCCCAGCAGACTCCAATGTGCCAGG
CTTGGGGGAACCCATTGCCCGAGCTCAAGTGTCTAAAGGA
TGGCACTTTCCCACTGCCCATCGGGGAATCAGTGACTGTC
ACTCGAGATCTTGAGGGCACCTACCTCTGTCGGGCCAGGA
GCACTCAAGGGGAGGTCACCCGCAAGGTGACCGTGAATGT
GCTCTCCCCCCGGTATGAGATTGTCATCATCACTGTGGTA
GCAGCCGCAGTCATAATGGGCACTGCAGGCCTCAGCACGT
ACCTCTATAACCGCCAGCGGAAGATCAAGAAATACAGACT
ACAACAGGCCCAAAAAGGGACCCCCATGAAACCGAACACA
CAAGCCACGCCTCCCTGA (SEQ ID NO:3)

### Example 15. Example of siRNA

In any one of Examples 11 to 14, the nucleic acid includes at least one sequences selected from the following:
CCAAUGUGCUAUUCAAACU **(SEQ ID NO:4)**
AGUUUGAAUAGCACAUUGG **(SEQ ID NO:5)**
GCCUCAGUCAGUGUGACCG **(SEQ ID NO:6)**
CGGUCACACUGACUGAGGC **(SEQ ID NO:7)**

### ICAM-1 Inhibitor 4 - CRISPR/Cas composition

### Example 16. Nuclease

CRISPR/Cas composition including the following:
a Cas protein, or a nucleic acid encoding the same; and
a guide RNA, or a nucleic acid encoding the same,
wherein the guide RNA is capable of interacting with the Cas protein to form a complex.

### Example 17. CRISPRi

CRISPRi composition including the following:
a CRISPRi protein fused with a Cas protein, and a gene expression inhibitory domain, or a nucleic acid encoding the CRISPRi protein; and
a guide RNA capable of targeting an ICAM-1 gene, or a nucleic acid encoding the same,
wherein the guide RNA is capable of interacting with the Cas protein to form a complex.

### Example 18. Specification of Cas protein

In Example 16 or 17, the Cas protein is selected from Cas9 and/or Cas12a proteins.

### Example 19. Specification of gene expression inhibitory domain

In Example 17, the gene expression inhibitory domain is at least one selected from βKRAB, DNMT, MeCP2, HDAC, LSD, SRDX, SALL1, and SDS3.

### ICAM-1 Inhibitor 5 - substances that inhibit ICAM-1 protein

### Example 20. General inhibitor

The ICAM-1 inhibitor may be any one selected from the following: compounds; small molecules; and aptamers, wherein the ICAM-1 inhibitor binds to the ICAM-1 protein, and is capable of preventing, inhibiting, and/or blocking interaction between the ICAM-1 protein and other molecules.

### Therapeutic mechanism for fibrosis

### Example 21. Therapeutic mechanism for fibrosis (1) - inactivation of myofibroblasts

Deactivating and/or inactivating myofibroblasts in fibrotic tissues in any one of Examples 1 to 4.

### Example 22. Specification of deactivation

In Example 21, deactivating and/or inactivating the myofibroblasts included at least one selected from the following:
(a) decomposing, reducing, degrading, lowering, removing, and/or decreasing extracellular matrix secreted by myofibroblasts;
(b) inhibiting, reducing, blocking, suppressing, and/or decreasing production of extracellular matrix by myofibroblasts; and
(c) reducing, killing, removing, and/or decreasing myofibroblasts in fibrotic tissues.

### Example 23. Specification of extracellular matrix - specification of functions

In Example 22, the extracellular matrix is fibrillar connective tissue.

### Example 24. Specification of extracellular matrix

In Example 22, the extracellular matrix may be at least one selected from collagen, fibronectin, hyaluronic acid, versican, and osteopontin (OPN).

### Example 25. Example of extracellular matrix degradation mechanism

In any one of Examples 22 to 24, (a) refers to one or more of the following, being promoted, activated, triggered, induced, and/or caused to function:
an increase in expression level of matrix metalloproteinase (MMP); and
a decrease in expression level of tissue inhibitors of metalloproteinase (TIMP).

### Example 26. Extracellular matrix-related markers

In any one of Examples 22 to 25, (b) refers to inhibiting, reducing, blocking, suppressing, and/or decreasing expression level of at least one selected from collagen (col1a1), osteopontin (OPN), versican (VER), fibronectin (FN), and hyaluronan synthase 3 (HAS3) mRNAs in tissues.

### Example 27. Myofibroblast-related markers

In any one of Examples 22 to 26, (c) refers to inhibiting, reducing, blocking, suppressing, and/or decreasing expression level of at least one selected from α-SMA, E-cadherin, Snail1, Snail2, Zeb1, Zeb2, Vimentin, N-cadherin, and Fibronectin in tissues.

### Example 28. Reversion of myofibroblasts to epithelial cells

In any one of Examples 22 to 26, (c) is induced by reversion of myofibroblasts to epithelial cells or fibroblasts.

### Example 29. Reversion of myofibroblasts to residential fibroblasts

In any one of Examples 22 to 26, (c) is induced by reversion of myofibroblasts to residential fibroblasts.

### Example 30. Therapeutic mechanism for fibrosis (2) - extracellular matrix degradation

In fibrotic tissues in any one of Examples 1 to 4,
blocking, inhibiting, suppressing, and/or relieving accumulation of extracellular matrix; and/or
decomposing, reducing, degrading, lowering, removing, and/or decreasing accumulated extracellular matrix.

### Example 31. Therapeutic mechanism for fibrosis (3) - ICAM-1-mediated signaling

In fibrotic tissues in any one of Examples 1 to 4, inhibiting, reducing, blocking, suppressing, and/or decreasing ICAM-1-mediated signaling.

### Example 32. Therapeutic mechanism for fibrosis (4) - TGF-β-mediated signaling inhibition

In fibrotic tissues in any one of Examples 1 to 4, inhibiting, reducing, blocking, suppressing, and/or decreasing TGF-β-mediated signaling, and/or a downstream signaling mechanism of TGF-β-mediated signaling.

### Example 33. Specification of downstream signaling mechanisms

In Example 32, the TGF-β-mediated signaling includes activation of Smad3 and/or Stat3 as downstream signaling mechanism.

### Example 34. Specification of cytokines

In Example 32 or 33, inhibiting, reducing, blocking, suppressing, and/or decreasing the TGF-β-mediated signaling, and/or the downstream signaling mechanism of the TGF-β-mediated signaling involves inhibiting, reducing, blocking, suppressing, and/or decreasing expression level of at least one protein selected from TGF-β, CTGF, IL11, TNFα, and IL8, and/or genes thereof.

### Pharmaceutical composition for treating fibrosis

### Example 35. Pharmaceutical composition

The pharmaceutical composition includes the following ingredients:
a therapeutically effective amount of ICAM-1 inhibitor; and
pharmaceutical acceptable carriers.

### Example 36. Specification of ICAM-1 inhibitor

In Example 35, the ICAM-1 inhibitor is at least one selected from the ICAM-1 neutralizing antibody in any one of Examples 5 to 8, the fragments of the ICAM-1 neutralizing antibody in Example 9 or 10, the ICAM-1 mRNA-silencing nucleic acid in any one of Examples 11 to 15, and the CRISPR/Cas composition in any one of Examples 16 to 19.

### Example 37. Specification of pharmaceutically acceptable carriers

In Example 35 or 36, the pharmaceutically acceptable carriers are at least one selected from the following:
binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol (PEG) wax; sweeteners; flavoring agents; syrups; liquid carriers such as fatty oils; sterile aqueous solutions; propylene glycol; polyethylene glycol; injectable esters such as ethyl oleate; suspending agents; emulsifiers; lyophilized preparations; topical formulations; stabilizers; buffers; animal oils; vegetable oils; waxes; paraffin; starches; tragacanth; cellulose derivatives; polyethylene glycol; silicone; bentonite; silica; talc; zinc oxide; and appropriate combinations thereof.

### Method of treating fibrosis

### Example 38. Method of treating fibrosis

A method of preventing, and/or treating diseases, disorders, and/or symptoms described in any one of Examples 1 to 4, comprising the following:
administering, injecting, introducing, and/or delivering a therapeutically effective amount of the ICAM-1 inhibitor, or the pharmaceutical composition in any one of Examples 35 to 37, to the subject,
wherein the ICAM-1 inhibitor is at least one selected from the ICAM-1 neutralizing antibody in any one of Examples 5 to 8, the fragments of the ICAM-1 neutralizing antibody in Example 9 or 10, the ICAM-1 mRNA-silencing nucleic acid in any one of Examples 11 to 15, and the CRISPR/Cas composition in any one of Examples 16 to 19.

### Example 39. Therapeutic mechanism (1)

In Example 38, the ICAM-1 inhibitor is capable of achieving, inducing, causing, and/or performing any one selected from Examples 21 to 29.

### Example 40. Therapeutic mechanism (2)

In Example 38, the ICAM-1 inhibitor is capable of achieving, inducing, causing, and/or performing Example 30.

### Example 41. Therapeutic mechanism (3)

In Example 38, the ICAM-1 inhibitor is capable of achieving, inducing, causing, and/or performing Example 31.

### Use of ICAM-1 Inhibitor for treating fibrosis

### Example 42. Use of ICAM-1 Inhibitor

The ICAM-1 inhibitor for preventing, and/or treating diseases, disorders, and/or symptoms in any one of Examples 1 to 4.

### Example 43. Specification of ICAM-1 Inhibitor

In Example 42, the ICAM-1 inhibitor is at least one selected from the ICAM-1 neutralizing antibody in any one of Examples 5 to 8, the fragments of the ICAM-1 neutralizing antibody in Example 9 or 10, the ICAM-1 mRNA-silencing nucleic acids in any one of Examples 11 to 15, and the CRISPR/Cas composition in any one of Examples 16 to 19.

### Example 44. Specification of therapeutic mechanism

In Example 42 or 43, the ICAM-1 inhibitor is capable of achieving, inducing, causing, and/or performing any one selected from Examples 21 to 29, Example 30, Example 31, and/or any one selected from Examples 32 to 34.

### Example 45. Use Claim

Use of the ICAM-1 inhibitor for preventing, and/or treating diseases, disorders, and/or symptoms in any one of Examples 1 to 4.

### Example 46. Specification of ICAM-1 inhibitor

In Example 45, the ICAM-1 inhibitor is at least one selected from the ICAM-1 neutralizing antibody in any one of Examples 5 to 8, the fragments of the ICAM-1 neutralizing antibody in Example 9 or 10, the ICAM-1 mRNA-silencing nucleic acid in any one of Examples 11 to 15, and the CRISPR/Cas composition in any one of Examples 16 to 19.

### Example 47. Specification of therapeutic mechanism

In Example 45 or 46, the ICAM-1 inhibitor is capable of achieving, inducing, causing, and/or performing any one selected from Examples 21 to 29, Example 30, Example 31, and/or any one selected from Examples 32 to 34.

### Use of ICAM-1 inhibitor for preparing fibrosis treatment

### Example 48. Use of ICAM-1 inhibitor for preparing fibrosis treatment

Use of the ICAM-1 inhibitor for preparing a pharmaceutical for treating the diseases, conditions, and/or symptoms in any one of Examples 1 to 4.

### Example 49. Specification of ICAM-1 inhibitor

In Example 48, the ICAM-1 inhibitor is at least one selected from the ICAM-1 neutralizing antibody in any one of Examples 5 to 8, the fragments of the ICAM-1 neutralizing antibody in Example 9 or 10, the ICAM-1 mRNA-silencing nucleic acids in any one of Examples 11 to 15, and the CRISPR/Cas composition in any one of Examples 16 to 19.

### Example 50. Specification of therapeutic mechanism

In Example 48 or 49, the ICAM-1 inhibitor is capable of achieving, inducing, causing, and/or performing any one selected from Examples 21 to 29, Example 30, and/or Example 31.

### Experimental Examples

The disclosure provided by this specification will be further described in detail through the following Experimental Examples and embodiments. These embodiments are intended solely to illustrate the contents disclosed in this specification, and it will be obvious to those skilled in the art that the scope of the contents disclosed in this specification is not limited by these embodiments.

### Experimental Example 1. Experimental methods and materials

Experimental Example 1.1. Materials
bleomycin Sulfate (A10152) was purchased from AdooQ, and used in this experiment. Alpha smooth muscle actin (α-SMA), Collagen I, and an ICAM-1 antibody were purchased from Abcam (Cambridge, UK), and used in this experiment.

The mice used in this experiment were 7-week-old male C57BL/6 mice, weighing approximately 20±2 g. After confirming individuals monitoring data, and appearance by manufacturer, the mice were acclimatized in a quarantine room for about one week, and only healthy individuals were used in the experiment after observing general symptoms. Animal specifications were maintained according to the standards of Hanyang University Animal Laboratory in an SPF room with an auto controller, at a temperature of 22±1°C, humidity of 50±10%, and a lighting cycle of 12 hours of light, and 12 hours of darkness. Sterile feed was provided with sterile water.

### Experimental Example 1.2. Tissue staining

Pulmonary tissue fixed in 10% neutral formalin was paraffin-embedded, sectioned into 4 µm thick slices, and stained with hematoxylin and eosin (H&E), and immunostained (α SMA, collagen 1, ICAM-1) to observe tissue changes. Additionally, tissue sections were prepared, and stained with Sirius red to observe collagen accumulation.

### Experimental Example 1.3. Western blot analysis

For Western blot analysis, 20 µg of protein was electrophoresed on a 10% SDS-polyacrylamide gel. After protein separation, the protein was transferred to a nitrocellulose membrane at 100 V for 1 hour, washed with 1X PBST (2.67 mM potassium chloride, 1.47 mM potassium phosphate monobasic, 137.93 mM sodium chloride, 8.1 mM sodium phosphate dibasic anhydrous, 0.1% Tween 20), and blocked with 5% skim milk (BD, U.S.) for 30 minutes. A primary antibody was diluted 1:1,000 in 1X PBST, and incubated overnight at 4°C. The membrane where reaction proceeds, was washed with 1X PBST, then reacted with a secondary antibody diluted 1:10,000 in 1X PBST at ambient temperature for 1 hour. Proteins were detected using ECL solution (PerkinElmer, U.S.), and developed on AGFA film (Agfa, Belgium).

### Experimental Example 1.4. Analysis of gene expression level by qPCR

Total RNA was extracted from tissues using an RNA minikit (Qiagen, Inc.), and cDNA was synthesized using Accupower RT mix reagent (Bioneer Corp., Seoul, Korea). Real-time PCR was performed using FastStart Essential DNA Green Master (Roche, Indianapolis, IN, USA).

### Experimental Example 1.5. Immunofluorescence staining

Cultured cells were fixed in 4% neutral formalin, reacted with a primary antibody (α-SMA, collagen 1), and then reacted with fluorescently conjugated secondary antibodies. Fluorescence microscopy images were used to observe protein expression.

### Experimental Example 2. Analysis of ICAM-1 expression in pulmonary tissue of patients with pulmonary fibrosis

### Experimental Example 2.1. Analysis of NCBI data

Expression data of an ICAM-1 gene in pulmonary tissues from patients with idiopathic pulmonary fibrosis (IPF), and normal pulmonary tissues based on NCBI database was analyzed. Specifically, the pulmonary tissues from IPF patients and normal pulmonary tissues were divided into groups, and analyzed relative expression levels of the ICAM-1 gene (FIG. 1).

The results showed that expression level of the ICAM-1 gene were significantly higher in IPF patients than in normal individuals.

### Experimental Example 2.2. Comparison of pulmonary tissue samples from IPF patients, and normal pulmonary tissue samples

Forty-eight pulmonary tissue samples from IPF patients, and eight normal pulmonary tissue samples were obtained.

The ICAM-1 protein was stained in each pulmonary tissue sample, and tissue images were observed under a microscope (FIG. 2).

Observation of the tissue images revealed that expression level of the ICAM-1 protein was higher in IPF patients than in normal individuals.

Additionally, the percentage of IHC-positive cells in each pulmonary tissue samples from IPF patients and normal individuals ("IPF" and "Normal") was measured and plotted, and was calculated to find whether there are significant differences between the groups (FIG. 3).

The plot results showed that the percentage of ICAM-1 protein-positive cells in the IPF group was significantly higher than that in the Normal group.

Furthermore, the IHC scores were calculated for each pulmonary tissue sample from the IPF group, and the Normal group, plotted, and then calculated to find whether there are significant differences between the groups (FIG. 4).

The plot results showed that the protein expression score of ICAM-1 was significantly higher in the IPF group than in the Normal group.

### Experimental Example 3. Cell-based experiments to evaluate therapeutic effect on pulmonary fibrosis

### Experimental Example 3.1. Identification of pulmonary fibrosis biomarkers #1 - expression analysis of protein

BEAS2B cells, a normal human pulmonary epithelial cell line, were administered with PBS, bleomycin (BLM), or a combination of bleomycin, and an ICAM-1 antibody. After 36 hours, expression levels of α-SMA, Col1A1, and ICAM-1 proteins were analyzed.

Specifically, 1) Western blot analysis was performed to measure the expression levels of ICAM-1, α-SMA, and Col1A1 proteins (left panel of FIG. 5), and 2) immunofluorescence staining was conducted followed by acquiring images under a fluorescence microscopy to detect whether α-SMA and Col1A1 proteins are expressed or not (right panel of FIG. 5). In the staining, α-SMA protein was stained in red, and Col1A1 protein was stained in green.

As a result, it was confirmed that in an experimental group administered with an ICAM-1 antibody, the expression levels of α-SMA, Col1A1, and ICAM-1 proteins were decreased compared with those of a BLM group. Similarly, immunofluorescence staining also showed reduced expression levels of α-SMA and Col1A1 proteins in the ICAM-1 antibody-administered group compared with those in the BLM group.

These results indicate that administration of bleomycin induces pulmonary fibrosis in pulmonary epithelial cells, and administration of an ICAM-1 antibody has a preventive and/or therapeutic effect on the bleomycin-induced pulmonary fibrosis.

### Experimental Example 3.2. Confirmation of pulmonary fibrosis markers #2 - analysis of mRNA expression

BEAS2B cells, a human normal pulmonary epithelial cell line, were administered with PBS, bleomycin (BLM), or a combination of BLM and an ICAM-1 antibody, and after 36 hours, the expression levels of α-SMA, Col1A1, and ICAM-1 genes were measured (FIG. 6).

As a result, in BLM group, it was confirmed that the expression levels of α-SMA and Col1A1 genes were increased, and fibrosis was induced. In contrast, when co-administered with the ICAM-1 antibody, the expression of said genes was suppressed, confirming the therapeutic effect on fibrosis.

### Experimental Example 3.3. Confirmation of epithelial-mesenchymal transition markers

BEAS2B cells, a human normal pulmonary epithelial cell line, were administered with PBS, bleomycin (BLM), or a combination of BLM, and an ICAM-1 antibody, and after 36 hours, the expression levels of Fibronectin, Vimentin, and Slug proteins was analyzed via Western blotting (FIG. 7).

As a result, administration of BLM only increased the expression levels of Fibronectin, Vimentin, and Slug proteins, indicating development of epithelial-mesenchymal transition. However, when co-administered with the ICAM-1 antibody, the expression levels of said markers were significantly reduced.

This indicates that BLM induces epithelial-mesenchymal transition in pulmonary epithelial cells, and that ICAM-1 antibody administration can suppress the epithelial-mesenchymal transition, and may further revert epithelial-mesenchymal transited cells to normal epithelial cells.

### Experimental Example 3.4. Confirmation of TGF-β signaling mechanism

BEAS2B cells, a human normal pulmonary epithelial cell line, were administered with PBS, bleomycin (BLM), or a combination of BLM, and an ICAM-1 antibody, and after 36 hours, expression level of TGF-β protein, and activation of its downstream signal, Smad3 (p-Smad3) were analyzed via Western blotting (FIG. 8).

As a result, it was observed that administration of BLM only activated TGF-β and its downstream signaling mechanism, p-Smad3. However, when co-administered with the ICAM-1 antibody, activation of TGF-β, and its downstream signaling mechanism, p-Smad3 was suppressed.

This indicates that TGF-β, and its downstream signaling mechanism can be inhibited by the ICAM-1 antibody.

### Experimental Example 3.5. Confirmation of expression level of TGF-β gene

BEAS2B cells, a human normal pulmonary epithelial cell line, were administered with PBS, bleomycin (BLM), or a combination of BLM, and an ICAM-1 antibody, and after 36 hours, expression levels of TGF-β and CTGF genes were measured (FIG. 9).

As a result, it was observed that in cells administered with BLM only, the expression levels of TGF-β, CTGF, and IL11 genes were increased compared with those in normal cells. However, it was observed that when co-administered with the ICAM-1 antibody, the expression levels of TGF-β, CTGF, and IL11 genes were reduced to levels similar to those of a normal condition.

### Experimental Example 3.6. Confirmation of expression levels of genes related to extracellular matrix and matrix-degrading enzymes

BEAS2B cells, a human normal pulmonary epithelial cell line, were administered with PBS, bleomycin (BLM), or a combination of BLM, and an ICAM-1 antibody, and after 36 hours, the expression levels of extracellular matrix, osteopontin (OPN), versican, and Has3 genes, as well as a collagen-degrading enzyme gene (MMP13), and extracellular matrix-degrading enzyme inhibitor gene including TIMP1 and TIMP2 genes, were measured (FIGS. 10 and 11).

As a result, in a group administered with BLM only, the expression levels of extracellular matrix genes (OPN, versican, HAS3) were increased, expression level of a collagen-degrading enzyme gene (MMP13) was decreased, and expression levels of matrix-degrading enzyme inhibitor (TIMP-1, TIMP-2) genes were increased. These findings indicate that an environment conducive to extracellular matrix accumulation, i.e., an environment in which fibrosis is induced, is formed. In contrast, in the group co-administered with the ICAM-1 antibody, it was confirmed that expression level of extracellular matrix gene were decreased, expression level of the collagen-degrading enzyme gene was increased, and expression level of matrix-degrading enzyme inhibitor gene was decreased, thereby forming an environment that prevents extracellular matrix accumulation, and promotes its degradation.

### Experimental Example 3.7. Analysis of changes in hydroxyproline content

BEAS2B cells, a human normal pulmonary epithelial cell line, were administered with PBS, bleomycin (BLM), and a combination of BLM, and an ICAM-1 antibody. After 36 hours, changes in hydroxyproline content were analyzed. Specifically, collagen accumulation capacity was evaluated using a hydroxyproline content assay (FIG. 12).

As a result, in a group administered with BLM only, hydroxyproline content, an indicator of collagen accumulation, was increased, confirming formation of a fibrotic environment. In contrast, in a group co-administered with the ICAM-1 antibody, hydroxyproline content was markedly reduced to level similar to that of normal cells (PBS group).

These findings indicate that the ICAM-1 antibody has an inhibitory effect on collagen accumulation in normal pulmonary epithelial cells.

### Experimental Example 3.8. Analysis of changes in TGF-β Levels by ELISA Assay

BEAS2B cells, a human normal pulmonary epithelial cell line, were administered with PBS, bleomycin (BLM), or a combination of BLM, and an ICAM-1 antibody. After 36 hours, changes in TGF-β levels were analyzed. Specifically, an amount of TGF-β produced was measured using a TGF-β ELISA assay (FIG. 13).

As a result, in a group administered with BLM only, production of TGF-β was increased, confirming formation of a fibrotic environment. In contrast, in a group co-administered with the ICAM-1 antibody, production of TGF-β was reduced.

These findings indicate that the ICAM-1 antibody has an inhibitory effect on production of TGF-β, a key factor in induction of fibrosis.

### Experimental Example 4. Animal Experiment for evaluating therapeutic effect on pulmonary fibrosis

### Experimental Example 4.1. bleomycin-induced pulmonary fibrosis animal model

An animal model of bleomycin-induced pulmonary fibrosis was used for an animal experiment. Specifically, pulmonary fibrosis was induced by intratracheal administration of bleomycin (1 mg/kg) once into mice. Starting on day 5, ICAM-1 antibody was administered intraperitoneally four times at 2-day intervals. On day 14, necropsy was performed, and pulmonary tissues were excised (FIG. 14).

The experimental groups were divided as follows: i) a group injected with PBS only instead of BLM, and not administered ICAM-1 antibody (PBS group, corresponding to normal condition), ii) a group administered BLM but not ICAM-1 antibody (BLM group, corresponding to condition in which pulmonary fibrosis is induced), and iii) a group administered both BLM, and ICAM-1 antibody (BLM+ICAM-1 Ab group, experimental group). Each group consisted of 10 animals.

### Experimental Example 4.2. Histological analysis for therapeutic effect of ICAM-1 antibody on fibrosis #1

For representative pulmonary tissue samples from each group (PBS, BLM, and BLM+ICAM-1 Ab) on day 14, histological observation was performed using H&E staining, Sirius Red staining, Masson's trichrome staining, and polarized microscopy. To assess pulmonary tissue condition, H&E staining was performed and observed results (FIG. 15, top), and Fibrosis Score was determined (FIG. 16). To assess collagen accumulation, Sirius Red staining was performed and observed results (FIG. 15, bottom), and Fibrosis Area was determined (FIG. 17). To assess collagen accumulation, Masson's trichrome staining was also performed and observed results (FIG. 18), and polarized microscopy was performed to observe collagen cross-linking (FIGS. 19 and 20).

As shown in FIGS. 15 to 17, in a group administered an ICAM-1 antibody, both Fibrosis Score (H&E staining) and Fibrosis Area (Sirius Red staining) were statistically significantly reduced compared with those in a group administered BLM only.

Furthermore, in a BLM group, it was confirmed that pulmonary fibrosis has been induced, resulting in tissue deformation and collagen accumulation. In contrast, in a BLM+ICAM-1 Ab group, pulmonary tissues similar to those of the normal tissues (PBS group) were observed, and markedly reduced collagen accumulation was also observed.

These results indicate that when ICAM-1 antibody is administered to fibrotic pulmonary tissues, it has the therapeutic effect of reverting the tissues to their original state of tissues, and removing accumulated collagen.

### Experimental Example 4.3. Histological analysis for therapeutic effect of ICAM-1 antibody on fibrosis #2

For representative pulmonary tissue samples from each group (PBS, BLM, and BLM+ICAM-1 Ab) on day 14, α-SMA and Col1A1 proteins were subjected to immunohistochemical staining, in which the proteins were stained brown to observe their accumulation (FIG. 21).

Upon observation, accumulation of α-SMA and Col1A1 proteins was confirmed in the BLM group, indicating that pulmonary fibrosis was induced. In contrast, in the BLM+ICAM-1 Ab group, marked reduction in α-SMA and Col1A1 proteins was observed to level similar to that of normal tissues (PBS group).

This demonstrates that when ICAM-1 antibody is administered to fibrotic tissues: 1) myofibroblasts in tissues is reduced (as evidenced by decreased α-SMA), and 2) accumulated collagen is removed, thereby achieving the therapeutic effect on fibrosis.

### Experimental Example 4.4. Analysis of expression levels of pulmonary fibrosis associated genes

For representative pulmonary tissue samples from each group (PBS, BLM, and BLM+ICAM-1 Ab) on day 14, expression levels of α-SMA, Col1A1, and ICAM-1 genes were analyzed. Specifically, pulmonary tissues were collected from individual animals in each group (n=3), and the expression levels of α-SMA, Col1A1, and ICAM-1 genes were measured using q-PCR (FIGS. 22 to 24).

As a result, in a BLM+ICAM-1 Ab group, the expression levels of α-SMA, Col1A1, and ICAM-1 genes in tissues were all reduced compared with those in the BLM group. This indicates that administration of ICAM-1 antibody leads to the therapeutic effect on fibrosis through reduction of myofibroblasts, and suppression of collagen accumulation.

### Experimental Example 4.5. Analysis of expression levels of pulmonary fibrosis associated proteins

For representative pulmonary tissue samples from each group (PBS, BLM, and BLM+ICAM-1 Ab) on day 14, expression levels of α-SMA, Col1A1, and ICAM-1 proteins were analyzed. Specifically, pulmonary tissues from individual animals in each group (n = 3) were collected, and protein expression was assessed via Western blot analysis (FIG. 25).

As a result, in a BLM+ICAM-1 Ab group, the expression levels of α-SMA, Col1A1, and ICAM-1 proteins in tissues were all reduced compared with those in the BLM group. This indicates that administration of ICAM-1 antibody to fibrotic tissues leads to the therapeutic effect on fibrosis by reducing myofibroblasts, and eliminating accumulated collagen.

### Experimental Example 4.6. Mouse behavioral assessment

In cases where pulmonary fibrosis develops, mice exhibit a marked reduction in motor activity; thus mouse behavioral assessment can be used to evaluate fibrosis onset and the therapeutic effect.

Specifically, bleomycin (100 mg/kg) was administered intraperitoneally four times to induce pulmonary fibrosis in mice. Beginning on day 12, ICAM-1 antibody was administered intraperitoneally four times at 2-day intervals. Mouse behavior was then observed on days 10, 16, and 20 (FIG. 26).

Upon observation of mouse behavioral patterns in each group (PBS, BLM, and BLM+ICAM-1 Ab), in the BLM group, pulmonary fibrosis was induced, and mice clustered together, and exhibited severely diminished activity. In contrast, in the BLM+ICAM-1 Ab group, mice exhibited activity level comparable to that of the normal mice (PBS group). This serves as firm evidence that in mice administered ICAM-1 antibody, pulmonary fibrosis was effectively treated, and pulmonary function was reverted to normal.

### Experimental Example 5. Confirmational experiment of therapeutic mechanisms for pulmonary fibrosis

### Experimental Example 5.1. Confirmation of TGF-β signaling mechanism #1 - expression analysis of protein

In a BLM-induced pulmonary fibrosis animal model described in Experimental Example 4, animals were divided into the following groups: i) a group administered PBS instead of BLM, and not administered ICAM-1 antibody (PBS group, corresponding to normal condition), ii) a group administered BLM, but not administered ICAM-1 antibody (BLM group, corresponding to fibrotic condition), and iii) a group administered both BLM, and ICAM-1 antibody (BLM+ICAM-1 Ab group, experimental group). Then, whether expression levels of TGF-β protein was increased or not, and changes in activity of downstream signals, Smad3 and Stat3, were analyzed. Specifically, pulmonary tissues were collected from three individual animals (#1 to #3) in each group, and expression level of TGF-β protein, and whether Smad3 and Stat3 are activated or not was confirmed using Western blotting (FIG. 27).

As a result, the BLM+ICAM-1 Ab group exhibited reduction in expression level of TGF-β protein, as well as reduced activity of Smad3 and Stat3, compared with that in the BLM group.

This demonstrates that while TGF-β and its downstream signals, Smad3 and Stat3 proteins, are activated upon pulmonary fibrosis induction, administration of ICAM-1 antibody blocks this signaling mechanism, and exerts the therapeutic effect on fibrosis.

### Experimental Example 5.2. Confirmation of TGF-β signaling mechanism #1 - expression analysis of gene

Pulmonary tissues were collected from individual animals (#1 to #3) in each group of animal model experiment in Experimental Example 4.1, and expression levels of TGF-β and CTGF genes were measured using q-PCR (FIGS. 28 to 30).

As a result, the BLM+ICAM-1 Ab group showed reduced expression levels of TGF-β, CTGF, and IL11 genes in tissues compared with those in the BLM group.

### Experimental Example 5.3. Analysis of epithelial-mesenchymal transition (EMT)-related markers

Pulmonary tissues were extracted from three individual animals (#1 to #3) in each group of animal model experiment in Experimental Example 4.1, and the expression levels of Fibronectin and Vimentin proteins were analyzed using Western blotting (FIG. 31).

Additionally, on day 14, for representative pulmonary tissue samples from each group (PBS, BLM, and BLM+ICAM-1 Ab groups), accumulation of EMT-related markers, Fibronectin and Vimentin proteins, was assessed. Specifically, immunohistochemistry was performed on representative pulmonary tissue samples in each group to stain Fibronectin and Vimentin proteins brown, and whether the proteins are accumulated or not was observed under a microscope (FIG. 32).

As a result, in the BLM group, pulmonary fibrosis was induced, exhibited elevated expression levels of Fibronectin and Vimentin proteins (FIG. 31), and accumulation of Fibronectin and Vimentin was observed in tissues (FIG. 32). In contrast, it was observed that the BLM+ICAM-1 Ab group exhibited reduced expression levels of EMT-related markers, Fibronectin and Vimentin proteins, and the accumulation levels in tissues were similar to those of normal tissues (PBS group).

This indicates that administration of ICAM-1 antibody suppresses epithelial-mesenchymal transition in fibrotic tissue, and provides an effect of reverting myofibroblasts to normal epithelial cells.

### Experimental Example 5.4. Analysis of changes in hybroxyproline content

Pulmonary tissue samples were collected on day 14 from each group in the animal model experiment of Experimental Example 4.1, and changes in hydroxyproline content were analyzed. Specifically, collagen accumulation capacity was assessed using a hydroxyproline content assay (FIG. 33).

As a result, in a BLM group, it was observed that pulmonary fibrosis induced, thereby increasing hydroxyproline content, and an indicator of collagen accumulation. In contrast, the BLM+ICAM-1 Ab group showed a significant reduction in hydroxyproline content, to level comparable to that of normal tissues (PBS group).

This demonstrates that administration of ICAM-1 antibody to fibrotic tissues suppresses collagen accumulation.

### Experimental Example 5.5. Analysis of changes in TGF-β level via ELISA assay

On day 14, pulmonary tissue samples were collected from each group in the animal model experiment of Experimental Example 4.1, and changes in level of TGF-β were analyzed. Specifically, the level of TGF-β produced was analyzed via a TGF-β ELISA assay (FIG. 34).

As a result, in the BLM group, it was observed that pulmonary fibrosis was induced, and TGF-β production was markedly increased. Conversely, in the BLM+ICAM-1 Ab group, TGF-β level was significantly reduced to level comparable to that of normal tissues (PBS group).

This indicates that administration of ICAM-1 antibody to fibrotic tissue provides effect of suppressing production of TGF-β.

### Experimental Example 6. Cell-based experiment of therapeutic effect on skin fibrosis

### Experimental Example 6.1. Confirmation of skin fibrosis markers

Skin fibroblasts were administered with TGF-β for 48 hours to induce skin fibrosis, and changes in expression levels of fibrotic markers, α-SMA and Col1A1 proteins, as well as their genes, were observed. Expression level of each protein was assessed by Western blotting (FIG. 35, left). Additionally, α-SMA and Col1A1 proteins were visualized via immunofluorescence staining, with α-SMA proteins stained green, and Col1A1 proteins stained red, to confirm the expression levels of those proteins (FIG. 35, right).

Furthermore, experimental groups were divided into the following three groups: Cont group administered with PBS only, TGF-β group administered with TGF-β only, and TGF-β + ICAM-1 Ab group administered with both TGF-β and ICAM-1 antibody. For each group, the expression levels of α-SMA and Col1A1 proteins were analyzed using q-PCR (FIG. 36).

As a result, in the TGF-β group in which skin fibroblasts were administered with TGF-β, expression levels of α-SMA and Col1A1 proteins, as well as their genes, were found to be increased, indicating that fibrosis was induced by TGF-β administration. In contrast, in a group co-administered with ICAM-1 antibody, expression levels of both α-SMA and Col1A1 proteins, and their genes were significantly suppressed, showing results similar to those of a normal control (Cont) group. This suggests that administration of ICAM-1 antibody to fibrotic cells inhibits differentiation of myofibroblasts, secretion and accumulation of collagen, or reverts the fibrotic cells to normal cells.

### Experimental Example 6.2. Analysis of expression levels of TGF-β, CTGF, and inflammation-related cytokine genes

Skin fibroblasts were administered with TGF-β for 48 hours to induce skin fibrosis, and expression levels of inflammation-related cytokine genes were evaluated by q-PCR. Specifically, changes in expression levels of TGF-β, CTGF, and inflammation-related cytokines including TNF-α, IL8, and IL11 genes in skin fibroblasts were assessed (FIGS. 37 and 38).

As a result, treatment with TGF-β led to an increase in expression levels of TGF-β, CTGF, and inflammation-related cytokine genes including TNF-α, IL8, and IL11 genes in skin fibroblasts.

Conversely, when ICAM-1 antibody was co-administered, it was found that expression levels of TGF-β, CTGF, and inflammation-related cytokine genes including TNF-α, IL8, and IL11 genes were significantly suppressed.

These findings demonstrate that ICAM-1 antibody reduces expression level of TGF-β, a critical signaling activator in induction of skin fibrosis. As a result, the ICAM-1 antibody suppresses signaling of CTGF, and critical inflammatory cytokines such as TNF-α, IL8, and IL11, thereby providing the therapeutic effect on skin fibrosis.

### Experimental Example 7. Animal experiment of therapeutic effect on skin fibrosis

### Experimental Example 7.1. Animal experiment model

An animal experiment was conducted using a bleomycin-induced skin fibrosis treatment model. bleomycin (0.5 mg/mL) was subcutaneously injected three times per week for six weeks to induce skin fibrosis. Starting in week 4, ICAM-1 antibody (250 µg/kg) was intraperitoneally administered three times per week for three weeks (total nine administrations) (FIG. 39).

The experiment was conducted separately in three groups: an experimental group administered both bleomycin and ICAM-1 antibody, an experimental group administered bleomycin only, and a control group administered PBS only.

### Experimental Example 7.2. Confirmation of therapeutic effect on skin fibrosis #1 - histological observation

To confirm the therapeutic effect of ICAM-1 antibody on skin fibrosis, histological observation was performed on skin tissue from each group in animal model experiment described in Experimental Example 7.1, to assess skin tissue morphology, and collagen accumulation.

Specifically, skin tissues from mice in each group was subjected to H&E staining, and observed under a microscope to assess skin tissue morphology (FIG. 40), and thickness of skin tissue was measured (FIG. 41). In addition, collagen in mouse skin tissue from each group was stained using Masson's trichrome staining, and observed under a microscope (FIG. 42). Furthermore, α-SMA and Col1A1 proteins in mouse skin tissues from each group were stained brown via immunohistochemical staining, and observed under a microscope (FIG. 43).

As a result, mouse skin tissues from the BLM group (in which skin fibrosis was induced) showed dermal thickening, altered distribution of tissue organelles, collagen accumulation, and accumulation of α-SMA and Col1A1 proteins, confirming that skin fibrosis had induced.

In contrast, the BLM+ICAM-1 Ab group (in which ICAM-1 antibody was administered) showed dermal thinning to level comparable to that of normal tissue (PBS group), restoration of subcutaneous fat layer, significantly reduced collagen accumulation, and marked reduction in accumulation of α-SMA and Col1A1 proteins to level comparable to that of normal tissues.

These results indicate that administration of ICAM-1 antibody to skin fibrotic tissues exhibits the therapeutic effect on fibrosis, and of reverting skin fibrotic tissues to normal tissues.

### Experimental Experiment 7.3. Confirmation of therapeutic effect on skin fibrosis #2 - observation of hair regrowth

In an experiment similar to Experimental Example 7.2, a bleomycin-induced skin fibrosis animal model experiment was performed (FIG. 44, top). As a result, it was observed that intraperitoneal administration of bleomycin in mice led to hair loss at an injection site (FIG. 44, bottom center; BLM group).

However, when ICAM-1 antibody was administered (FIG. 44, bottom right; BLM + ICAM-1 Ab group), the previously hairless area showed reversion to a normal hair-covered state.

This serves as an evidence that ICAM-1 antibody exhibits the therapeutic effect on skin fibrosis.

### Experimental Example 8. Cell-based experiment of therapeutic effect on hepatic fibrosis

### Experimental Example 8.1. Confirmation of hepatic fibrosis markers

For cell-based experiments on hepatic fibrosis, a human hepatic stellate cell line LX-2 was used. LX-2 cells were divided into three groups, 1) control group (Cont) administered with PBS only, 2) comparative group administered with TGF-β only to induce fibrosis (TGF-β), and 3) experimental group administered with both TGF-β and ICAM-1 antibody (TGF-β+ICAM-1 Ab), and administered with each reagent.

For each group, expression levels of α-SMA and Col1A1 proteins were analyzed by Western blotting (FIG. 45, left). In addition, α-SMA and Col1A1 proteins were visualized via immunofluorescence staining, in which α-SMA was stained green, and Col1A1 was stained red (FIG. 45, right). Cell samples were collected from each group, and further subjected to q-PCR to analyze expression levels of α-SMA, Col1A1, ICAM-1, TGF-β, and CTGF genes (FIGS. 46 and 47).

As a result, the comparative group administered with TGF-β only exhibited increased expression levels of α-SMA and Col1A1 proteins and their genes, indicating induction of fibrosis. Specifically, it was found that the LX-2 cells had differentiated into myofibroblasts, and expressed and secreted collagen, which accumulated in the tissues. In contrast, in the experimental group co-administered with ICAM-1 antibody, expression levels of α-SMA and Col1A1 proteins and their genes were significantly suppressed to level comparable to that of the control group. Additionally, reduced expression levels of TGF-β and CTGF genes were also confirmed.

These results indicate that the ICAM-1 antibody has the therapeutic effect on fibrosis. Specifically, the ICAM-1 antibody reduces myofibroblasts in fibrotic tissues, inhibits expression and secretion of collagen in the tissues, prevents collagen accumulation in the tissues, and facilitates removal of accumulated collagen.

### Experimental Example 9. Confirmation of ICAM-1 expression suppressive effect by ICAM-1-targeting siRNA

An experiment was conducted to evaluate whether ICAM-1-targeting siRNA suppresses ICAM-1 expression.

Normal pulmonary fibroblasts were administered with ICAM-1-targeting siRNA at a concentration of 100 nM to inhibit its gene expression, and whether ICAM-1 protein expression was also inhibited or not was subsequently evaluated using Western blotting (FIG. 48).

mRNA sequence of a human ICAM-1 gene is as follows: 
CGCTGAATGGGGTTCCAGCCCAGCCACTGGGCCCGAGGGC
CCAGCTCCTGCTGAAGGCCACCCCAGAGGACAACGGGCGC
AGCTTCTCCTGCTCTGCAACCCTGGAGGTGGCCGGCCAGC
TTATACACAAGAACCAGACCCGGGAGCTTCGTGTCCTGTA
TGGCCCCCGACTGGACGAGAGGGATTGTCCGGGAAACTGG
ACGTGGCCAGAAAATTCCCAGCAGACTCCAATGTGCCAGG
CTTGGGGGAACCCATTGCCCGAGCTCAAGTGTCTAAAGGA
TGGCACTTTCCCACTGCCCATCGGGGAATCAGTGACTGTC
ACTCGAGATCTTGAGGGCACCTACCTCTGTCGGGCCAGGA
GCACTCAAGGGGAGGTCACCCGCAAGGTGACCGTGAATGT
GCTCTCCCCCCGGTATGAGATTGTCATCATCACTGTGGTA
GCAGCCGCAGTCATAATGGGCACTGCAGGCCTCAGCACGT
ACCTCTATAACCGCCAGCGGAAGATCAAGAAATACAGACT
ACAACAGGCCCAAAAAGGGACCCCCATGAAACCGAACACA
CAAGCCACGCCTCCCTGA (SEQ ID NO:3)
,
 and siRNA used in Experimental Example 8 to silence the SEQ ID NO:3 is as follows:

**Table 1.**

| Gene name | Gene ID/accession number | siRNA name | siRNA sequence (5' to 3') | SEQ ID NO |
|---|---|---|---|---|
| ICAM-1 | NM_000201.2 | siRNA-1 | CCAAUGUGCUAUUCAAACU | 4 |
| ICAM-1 | NM_000201.2 | siRNA-1' | AGUUUGAAUAGCACAUUGG | 5 |
| ICAM-1 | NM_000201.2 | siRNA-2 | GCCUCAGUCAGUGUGACCG | 6 |
| ICAM-1 | NM_000201.2 | siRNA-2' | CGGUCACACUGACUGAGGC | 7 |

As a result, the ICAM-1-targeting siRNA used in this experiment effectively suppressed expression of the ICAM-1 protein when administered into cells. When expression level of the ICAM-1 protein is reduced, inhibition or reduction of ICAM-1 function will occur. Therefore, similar to results of the ICAM-1 neutralizing antibody experiments in Experimental Examples 1 to 7, administration of ICAM-1-targeting siRNA to fibrotic cells, and/or tissues is expected to exhibit the therapeutic effect on fibrosis, comparable to that observed with administration of the ICAM-1 neutralizing antibody.

### Experimental Example 10. Confirmation of therapeutic effect on fibrosis by ICAM-1-targeting siRNA

### Experimental Example 10.1. Confirmation of therapeutic effect on human pulmonary fibroblasts

With reference to Experimental Examples 3 and 9, the therapeutic effect on pulmonary fibrosis by ICAM-1-targeting siRNA was confirmed.

Specifically, human pulmonary fibroblasts were administered with control siRNA with random sequences (hereinafter, si-cont), bleomycin and si-cont (BLM+si-cont), and bleomycin and ICAM-1-targeting siRNA (BLM + si-ICAM-1), respectively. After 48 hours, the following measurements were taken, wherein
the ICAM-1-targeting siRNA has RNA sequence 5'-CCAAUGUGCUAUUCAAACU-3' (SEQ ID NO: 4):
1) measurement of changes in hydroxyproline content using a hydroxyproline content assay (Fig. 49);
2) measurement of TGF-β concentration using a TGF-β ELISA assay (Fig. 50); and
3) measurement of expression levels of ICAM-1, α-SMA, Col1A1, and TGF-β mRNAs (Fig. 51).

The results showed that, similar to an administration of an ICAM-1 antibody, 1) hydroxyproline content was reduced to level comparable to that of normal cells (si-cont); 2) production of TGF-β was inhibited; and 3) expression levels of fibrosis-associated markers α-SMA and Col1A1 were suppressed.

Therefore, ICAM-1-targeting siRNA is also considered to exert preventive/therapeutic effects on fibrosis in human pulmonary cells, similarly to those achieved by the ICAM-1 antibody.

### Experimental Example 10.2: Confirmation of therapeutic effect on fibrosis in human dermal fibroblasts

With reference to Experimental Examples 6 and 9, the therapeutic effect on dermal fibrosis by the ICAM-1-targeting siRNA was confirmed.

Specifically, human dermal fibroblasts were administered with control siRNA with random sequences (hereinafter, si-cont), bleomycin and si-cont (BLM+si-cont), and bleomycin and ICAM-1-targeting siRNA (BLM + si-ICAM-1), respectively. After 48 hours, expression levels of ICAM-1, α-SMA, Col1A1, and TGF-β mRNAs were assessed (Fig. 52).

The ICAM-1-targeting siRNA has a RNA sequence 5'-CCAAUGUGCUAUUCAAACU-3' (SEQ ID NO: 4)
As a result, it was confirmed that similar to administration of an ICAM-1 antibody, expression levels of fibrosis-associated markers α-SMA, Col1A1, and TGF-β were suppressed.

Therefore, ICAM-1-targeting siRNA is also considered to exert preventive/therapeutic effects on fibrosis in human pulmonary cells, similarly to an ICAM-1 antibody.

### Industrial Applicability

The present disclosure provides a fibrosis treatment, the treatment containing the ICAM-1 inhibitor, and its use, as well as a method of treating fibrosis using the ICAM-1 inhibitor. The ICAM-1 inhibitor, and the method of treating fibrosis using the same, provided in the present disclosure, can be used to prevent or treat fibrosis regardless of types of fibrotic tissues.

## Claims

1. An ICAM-1 neutralizing antibody for treating fibrosis.

2. The ICAM-1 neutralizing antibody of Claim 1, wherein the antibody is capable of neutralizing ICAM-1 having an amino acid sequence of

MAPSSPRPALPALLVLLGALFPGPGNAQTSVSPSKV
ILPRGGSVLVTCSTSCDQPKLLGIETPLPKKELLLPGNNR
KVYELSNVQEDSQPMCYSNCPDGQSTAKTFLTVYWTPERV
ELAPLPSWQPVGKNLTLRCQVEGGAPRANLTVVLLRGEKE
LKREPAVGEPAEVTTTVLVRRDHHGANFSCRTELDLRPQG
LELFENTSAPYQLQTFVLPATPPQLVSPRVLEVDTQGTVV
CSLDGLFPVSEAQVHLALGDQRLNPTVTYGNDSFSAKASV
SVTAEDEGTQRLTCAVILGNQSQETLQTVTIYSFPAPNVI
LTKPEVSEGTEVTVKCEAHPRAKVTLNGVPAQPLGPRAQL
LLKATPEDNGRSFSCSATLEVAGQLIHKNQTRELRVLYGP
RLDERDCPGNWTWPENSQQTPMCQAWGNPLPELKCLKDGT
FPLPIGESVTVTRDLEGTYLCRARSTQGEVTRKVTVNVLS
PRYEIVIITVVAAAVIMGTAGLSTYLYNRQRKIKKYRLQQ
AQKGTPMKPNTQATPP (SEQ ID NO:1)
.

3. The ICAM-1 neutralizing antibody of claim 2, wherein an epitope of the antibody has an amino acid sequence of

QTSVSPSKVILPRGGSVLVTCSTSCDQPKLLGIETPLPKKELLLPGNNRK
VYELSNVQEDSQPMCYSNCPDGQSTAKTFLTVYWTPERVE
LAPLPSWQPVGKNLTLRCQVEGGAPRANLTVVLLRGEKEL
KREPAVGEPAEVTTTVLVRRDHHGANFSCRTELDLRPQGL
ELFENTSAPYQLQTFVLPATPPQLVSPRVLEVDTQGTVVC
SLDGLFPVSEAQVHLALGDQRLNPTVTYGNDSFSAKASVS
VTAEDEGTQRLTCAVILGNQSQETLQTVTIYSFPAPNVIL
TKPEVSEGTEVTVKCEAHPRAKVTLNGVPAQPLGPRAQLL
LKATPEDNGRSFSCSATLEVAGQLIHKNQTRELRVLYGPR
LDERDCPGNWTWPENSQQTPMCQAWGNPLPELKCLKDGTF
PLPIGESVTVTRDLEGTYLCRARSTQGEVTRKVTVNVLSPRYE (SEQ ID NO:2)
.

4. The ICAM-1 neutralizing antibody of any one of claims 1 to 3, wherein the fibrosis is selected from the group consisting of:
hypertrophic scar; systemic sclerosis; multiple cancers; pulmonary arterial hypertension; glial scar; Alzheimer's disease; cardiac fibrosis; hypertrophic cardiomyopathy; cardiac dysfunction; valvular disease; arrhythmia; myelofibrosis; myelodysplastic syndrome; chronic myelogenous leukemia; cirrhosis; portal hypertension; hepatocellular carcinoma; nonalcoholic steatohepatitis (NASH); intestinal fibrosis; enteropathies; inflammatory bowel disease; arthrofibrosis; subretinal fibrosis; epiretinal fibrosis; vision loss; idiopathic pulmonary fibrosis; cystic fibrosis; pulmonary hypertension; thromboembolic disease; emphysema; renal fibrosis; cystic fibrosis; nephrogenic systemic fibrosis; chronic kidney disease; renal anemia; retroperitoneal fibrosis; mediastinal fibrosis; pancreatic fibrosis; chronic pancreatitis; duct obstruction; autoimmune-interstitial pulmonary disease such as ankylosing spondylitis, and rheumatoid arthritis; connective tissue disease-interstitial pulmonary disease such as rheumatoid arthritis-interstitial pulmonary disease; and non-idiopathic pulmonary fibrosis-interstitial pulmonary disease.

5. The ICAM-1 neutralizing antibody of any one of claims 1 to 4, wherein the antibody induces degradation of an extracellular matrix accumulated in a fibrotic tissue.

6. The ICAM-1 neutralizing antibody of claim 5, wherein the extracellular matrix accumulated in the fibrotic tissue is a collagen.

7. The ICAM-1 neutralizing antibody of any one of claims 1 to 6, wherein the antibody inactivates myofibroblasts in the fibrotic tissue.

8. The ICAM-1 neutralizing antibody of claim 7, wherein the inactivating myofibroblasts in the fibrotic tissue refers to at least one selected from the following:
(a) degrading an extracellular matrix secreted by the myofibroblast;
(b) inhibiting secretion of an extracellular matrix by the myofibroblast; and
(c) reducing myofibroblasts in the fibrotic tissue.

9. The ICAM-1 neutralizing antibody of claim 8, wherein the extracellular matrix of the (a) and (b) is a collagen.

10. The ICAM-1 neutralizing antibody of claim 8 or claim 9, wherein the (b) induces reduction in the expression level of the COL1A1 gene.

11. The ICAM-1 neutralizing antibody of any one of claims 8 to 10, wherein the (c) induces reduction in the expression level of the α-SMA gene.

12. The ICAM-1 neutralizing antibody of any one of claims 8 to 10, wherein the (c) occurs by reversion of the myofibroblast into epithelial cell of fibroblast.

13. The ICAM-1 neutralizing antibody of any one of claims 1 to 12, wherein the antibody inhibits ICAM-1-mediated signaling in the fibrotic tissue.

14. The ICAM-1 neutralizing antibody of claim 13, wherein the ICAM-1-mediated signaling is selected from the group consisting of:
(a) epithelial-to-mesenchymal transition (EMT); and
(b) extracellular matrix remodeling.

15. A method for preventing or treating a fibrosis, comprising:
administering a therapeutically effective amount of an ICAM-1 neutralizing antibody of any one of claims 1 to 14 to s subject.

16. A pharmaceutical composition for treating a fibrosis, comprising:
a therapeutically effective amount of the ICAM-1 neutralizing antibody of any one of claims 1 to 14; and
a pharmaceutically acceptable carrier.

17. The pharmaceutical composition of claim 16, wherein the pharmaceutically acceptable carrier is at least one selected from the group consisting of:
binders such as lactose, saccharose, sorbitol, mannitol, starch, amylopectin, cellulose, or gelatin; excipients such as dicalcium phosphate; disintegrants such as corn starch or sweet potato starch; lubricants such as magnesium stearate, calcium stearate, sodium stearyl fumarate, or polyethylene glycol (PEG) wax; sweeteners; flavoring agents; syrups; liquid carriers such as fatty oils; sterile aqueous solutions; propylene glycol; polyethylene glycol; injectable esters such as ethyl oleate; suspending agents; emulsifiers; lyophilized preparations; topical formulations; stabilizers; buffers; animal oils; vegetable oils; waxes; paraffin; starches; tragacanth; cellulose derivatives; polyethylene glycol; silicone; bentonite; silica; talc; zinc oxide; and appropriate combinations thereof.

18. A use of the ICAM-1 neutralizing antibody of any one of claims 1 to 14 in manufacture of a medicament for treating fibrosis.
